# FASCICULE DE BREVET EUROPEEN

(11) **EP 1 618 927 B1**
(45) Date de publication et mention de la délivrance du brevet: **01.08.2012**
(21) Numéro de dépôt: 05291123.7
(22) Date de dépôt: 25.05.2005
(51) Int. Cl.: A61Q 17/04, A61K 8/49, A61K 8/37, A61K 8/42, A61K 8/92, A61Q 19/04, A61Q 19/00, A61Q 5/00, A61Q 1/00

(54) **Compositions photoprotectrices contenant un filtre triazine solubilisé par un mélange d'un dérivé arylalkyl benzoate et d'une huile amidée ; utilisations en cosmétique**
Lichtschutzzubereitungen enthaltend einen Tiazinefilter, das mit einer Mischung aus arylalkyl benzoate und Amid-Gruppe enthaltende Öl solubilisiert wird; Verwendungen in Kosmetik
Sunscreen compositions containing a triazine filter solubilised by a mixture of an arylalkyl benzoate derivative and an amide-modified oil; uses in cosmetics

(30) Priorité: 02.07.2004 FR 0451421
(43) Date de publication de la demande: 25.01.2006
(73) Titulaire: L'Oréal, 75008 Paris (FR)
(72) Inventeur: Candau, Didier, 91570 Bievres (FR); Fiandino, Cécile, 75013 Paris (FR)
(74) Mandataire: Miszputen, Laurent

(56) Documents cités:
- EP-A- 0 748 623
- EP-A- 1 269 980
- WO-A-03/039510
- WO-A-2005/009341
- US-A1- 2005 019 280
- US-B1- 6 355 264

## Description

La présente invention concerne une composition photoprotectrice comprenant au moins un dérivé de 1,3,5 triazine solubilisé par mélange constitué d'au moins un dérivé arylalkyl benzoate et d'au moins une huile amidée ainsi que ses diverses utilisations cosmétiques notamment la protection de la peau et/ou des lèvres et/ou des phanères contre le rayonnement ultra-violet, en particulier le rayonnement solaire.

On sait que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel; ce rayonnement UV-B doit donc être filtré.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photo-allergiques. Il est donc souhaitable de filtrer aussi le rayonnement UV-A.

De nombreuses compositions cosmétiques destinées à la photoprotection (UV-A et/ou UV-B) de la peau ont été proposées à ce jour.

Ces compositions anti-solaires se présentent assez souvent sous la forme d'une émulsion de type huile-dans-eau (c'est à dire un support cosmétiquement acceptable constitué d'une phase continue dispersante aqueuse et d'une phase discontinue dispersée huileuse) qui contient, à des concentrations diverses, un ou plusieurs filtres organiques classiques, lipophiles et/ou hydrophiles, capables d'absorber sélectivement les rayonnements UV nocifs, ces filtres (et leurs quantités) étant sélectionnés en fonction du facteur de protection solaire recherché, le facteur de protection solaire (FPS) s'exprimant mathématiquement par le rapport de la dose de rayonnement UV nécessaire pour atteindre le seuil érythématogène avec le filtre UV sur la dose de rayonnement UV nécessaire pour atteindre le seuil érythématogène sans filtre UV.

Les dérivés de 1,3,5-triazine sont particulièrement recherchés dans la cosmétique solaire du fait qu'ils sont fortement actifs dans l'UVB, et même dans l'UV-A pour certains de ces composés selon la nature des substituants mis en jeu. De plus, ils sont photostables, c'est à dire qu'ils se dégradent peu ou pas chimiquement sous l'action du rayonnement UV. Ils sont notamment décrits dans les demandes de brevet US 4367390, EP863145, EP517104, EP570838, EP796851, EP775698, EP878469 et EP933376, et on connaît en particulier :
- la 2,4,6-tris[p-(2'-éthylhexyl-1'-oxycarbonyl)anilino]-1,3,5-triazine ou « Ethylhexyl Triazone » (nom INCI), vendue sous la dénomination commerciale « Uvinul T 150 » par la société BASF,
- la 2-[(p-(tertiobutylamido)anilino]-4,6-bis-[(p-(2'-éthylhexyl-1'-oxycarbonyl)anilino]-1,3,5-triazine ou « Diethylhexyl Butamido Triazone » (nom INCI) vendu sous le nom commercial « UVASORB HEB » par SIGMA 3V,
- la 2,4-bis {[4-2-éthyl-hexyloxy)]-2-hydroxy]-phenyl}-6-(4-méthoxy-phenyl)-1,3,5-triazine ou « Bis-Ethylhexyloxyphenol Methoxyphenyl Triazine» (nom INCI) vendu sous le nom commercial «TINOSORB S » par CIBA SPECIALTY CHEMICALS.

Dans l'art antérieur, il a été proposé d'utiliser des dérivés de 1,3,5-triazine dans des huiles comme des esters et plus particulièrement des benzoates d'alkyles en C12-C15 ("FINSOLV TN" de chez Finetex), ou des triglycérides et notamment des triglycérides d'acides gras en C8-C12 ("MIGLYOL 812" de chez Hüls), ou encore des monoalcools ou des polyols gras oxyéthylénés ou oxypropylénés ( « CETIOL HE » de chez Henkel ou « WITCONOL AM» de chez WITCO). L'utilisation de ces huiles présentent deux inconvénients:
- soit l'apparition au cours du temps, de cristallisation dans les formules qui nuisent aux qualités cosmétiques, à la stabilité et à l'efficacité des produits solaires ;
- soit la limitation de la concentration en filtres dans les formules ce qui ne permet pas d'obtenir des produits suffisamment efficaces.

Le problème technique posé à la base de la présente invention est donc d'améliorer l'efficacité photoprotectrice, les propriétés cosmétiques et la stabilité des compositions contenant de tels dérivés de 1,3,5-triazine.

Dans cet objectif, on a déjà proposé dans le brevet EP0748623 d'ajouter à la 2,4,6-tris[p-(2'-éthylhexyl-1'-oxycarbonyl)anilino]-1,3,5-triazine ou «Ethylhexyl Triazone» des composés amidés N,N-disubstitués et sans propriétés émulsionnantes comme le N,N-diéthyl-3-méthylbenzamide ou le N-butyl, N-acétyl aminopropionate d'éthyle.

On a également également proposé dans le brevet EP1269980 d'ajouter à des filtres UV de type triazine des esters N-acylés d'acide aminés comme le N-lauroylsarcosinate d'isopropyle.

Les résultats obtenus avec ces huiles amidées ne sont pas encore pleinement satisfaisants.

De manière inattendue et surprenante, la demanderesse a découvert que l'on pouvait résoudre le problème technique évoqué ci-dessus en utilisant un mélange d'huile amidée et de dérivé arylalkyl benzoate dans une composition contenant au moins un dérivé de triazine, dans un quantité suffisante permettant de solubiliser à lui seul la quantité totale de dérivé de triazine.

Ce mélange particulier permet l'obtention de compositions anti-solaires stables contenant des dérivés 1,3,5 triazine et qui présentent un facteur de protection solaire supérieur à ceux des compositions de l'art antérieur contenant les dérivés 1,3,5 triazine et les huiles amidées. Ces compositions possèdent en outre des qualités cosmétiques améliorées. Elles permettent notamment une bonne hydratation de la peau c'est-à-dire que l'on n'observe pas de dessèchement de la peau, ni un toucher trop gras.

La présente invention a donc pour objet une composition cosmétique ou dermatologique photoprotectrice contenant
a) au moins un dérivé de 1,3,5-triazine et
b) au moins un mélange constitué d'au moins une huile présentant dans sa structure au moins un motif amide et d'au moins un dérivé arylalkyl benzoate.

D'autres objets de l'invention apparaîtront à la lecture de la description et des exemples qui suivent.

On entend par composition photoprotectrice, toute composition contenant au moins un composé organique et/ou au moins composé minéral susceptible de filtrer les radiations UV-A et/ou UV-B par phénomène d'absorption, réflexion ou de diffusion.

On entendra dans tout le texte de la description par «huile présentant dans sa structure au moins un motif amide » tout composé comportant dans sa structure chimique au moins un groupement (ou fonction) amide du type : 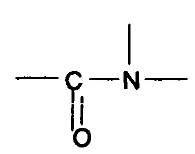 et présentant simultanément les caractéristiques suivantes :
a) liquide à 25 °C ,
b) insoluble ou non miscible à l'eau à 25 °C
c) ne présente pas de propriétés émulsionnantes.

De façon préférentielle, le mélange huile amidée/dérivé arylalkyl benzoate est présent dans la composition dans une quantité suffisante permettant de solubiliser à lui-seul (sans qu'il soit nécessaire d'utilser un autre solvant) la quantité de totale de filtre triazine présent dans la composition.

L'huile ou les huiles présentant dans leur structure au moins un motif amide conformes à l'invention sont choisies de préférence parmi les composés de formule (1) suivante : 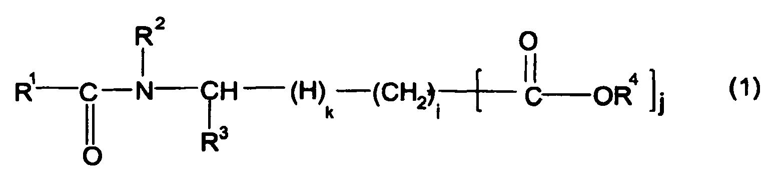 dans laquelle :
- le radical R¹ représente un radical hydrocarboné monovalent, saturé ou insaturé, aliphatique, cycloaliphatique ou cyclique, éventuellement fonctionnalisé, contenant de 1 à 30 atomes de carbone, de préférence de 1 à 22 atomes de carbone, bornes incluses ;
- les radicaux R² , R³ et R⁴ qui peuvent être identiques ou différents, représentent l'hydrogène ou des radicaux hydrocarbonés monovalents, saturés ou insaturés, aliphatiques, cycloaliphatiques ou cycliques, éventuellement fonctionnalisés, contenant de 1 à 30 atomes de carbone, de préférence de 1 à 22 atomes de carbone, bornes incluses;
- k vaut 0 ou 1 ;
- i est un entier de 0 à 2 ;
- j vaut 0 ou 1 ;
- sous réserve que :

- lorsque j = 1 alors k vaut 0 et lorsque j = 0 alors i =0 et k =1.

Comme exemples de radicaux hydrocarbonés saturés aliphatiques, on peut notamment citer les radicaux alkyles, linéaires ou ramifiés, substitués ou non, en C₁-C₃₀, de préférence en C₁-C₂₂, et en particulier les radicaux méthyle, éthyle, n-propyle, isopropyle, n-butyle, isobutyle, ter.-butyle, pentyle, n-amyle, isoamyle, néopentyle, n-hexyle, n-heptyle, n-octyle, 2-éthylhexyle, tert-octyle, décyle, lauryle et octadécyle.

A titre d'exemples de radicaux hydrocarbonés saturés cycliques, on peut notamment citer les radicaux cyclopentyle et cyclohexyle, éventuellement substitués, en particulier par des radicaux alkyles.

Comme exemples de radicaux hydrocarbonés insaturés aliphatiques, on peut notamment citer les radicaux alcényles ou alcynyles, linéaires ou ramifiés, substitués ou non, en C₂₋C₃₀, de préférence en C₂-C₂₂, et particulier les radicaux vinyle, allyle, oléyle et linoléyle.

Comme exemples de radicaux hydrocarbonés insaturés cycliques, on peut notamment citer les radicaux aryles tels que phényle et naphtyle, éventuellement substitués, en particulier par des alkyles, comme par exemple le radical tolyle, et à titre d'exemples de radicaux cycloaliphatiques insaturés, on peut citer plus particulièrement les radicaux benzyle et phényléthyle.

Par radicaux fonctionnalisés, on entend plus particulièrement des radicaux comportant dans leur structure chimique, tant dans la chaine principale que sur un chainon secondaire, un ou plusieurs groupements fonctionnels du type notamment esters, éthers, alcools, amines, amides et cétones, mais de préférence esters.

Les huiles amidées de formule (1) préférentielles sont choisies parmi celles où :
R¹ représente un radical alkyle en C₁-C₂₂, linéaire ou ramifiée ; un radical alcényle en C₂₋C₂₂, linéaire ou ramifié ; un radical aryle ;
R² représente un atome d'hydrogène ou un groupe alkyle en C₁-C₆, linéaire ou ramifiée ;
R³ représente un atome d'hydrogène ou un groupe alkyle en C₁-C₆, linéaire ou ramifiée ;
R⁴ représente un radical alkyle en C₁-C₁₀ linéaire ou ramifié, ou un radical alcényle en C₂₋C₁₀ linéaire ou ramifié ou un reste stérol.

Dans la formule (1) présentée ci-dessus, le groupement R¹(CO)- est un groupement acyle d'un acide choisi de préférence dans le groupe formé par l'acide acétique, l'acide toluique, l'acide caprique, l'acide laurique, l'acide myristique, l'acide palmitique, l'acide stéarique, l'acide béhénique, l'acide linoléique, l'acide linolénique, l'acide oléique, l'acide isostéarique, l'acide 2-éthylhexanoïque, les acides gras d'huile de coco, les acides gras d'huile de palmiste. Ces acides peuvent en outre présenter un groupe hydroxyle.

Dans la formule (1), lorsque j vaut 1, la partie -N(R²)CH(R³)(CH₂)q(CO)- de l'ester d'aminoacide est de préférence choisie parmi celles correspondant aux aminoacides suivants :
glycine, alanine, valine, leucine, isoleucine, sérine, thréonine, proline, hydroxyproline, β-alanine, N-butyl β-alanine, acide aminobutyrique, acide aminocaproïque, sarcosine, ou N-méthyl-β-alanine.

Dans la formule (1), lorsque j vaut 1, la partie des esters aminoacides correspondant au groupe OR⁴ peut être obtenue à partir des alcools choisis dans le groupe formé par le méthanol, éthanol, propanol, isopropanol, butanol, tert-butanol, isobutanol, 3-méthyl-1-butanol, 2-méthyl-1-butanol, pentanol, héxanol, cyclohéxanol, octanol, 2-éthylhéxanol, décanol, alcool laurylique, alcool myristique, alcool cétylique, alcool cétostéarylique, alcool stéarylique, alcool oléique, alcool béhénylique, alcool de jojoba, alcool 2-héxadécylique, alcool 2-octyldodécanol et alcool isostéarylique.

Les huiles présentant dans leur structure au moins une fonction amide de formule (1) conformes à l'invention sont connues en soi. Certaines sont notamment décrites avec leurs modes de préparation dans les demandes de brevets EP 1 044 676 et EP 0 928 608 de la société AJINOMOTO CO. D'autres sont connues en cosmétique comme insectifuges comme le N-acétyl N-butylaminopropionate ou le N,N-diéthyl-toluamide.

Parmi les composés de formule (1) particulièrement préférés, on peut citer :
le N-acetyl N-butylaminopropionate de formule suivante : 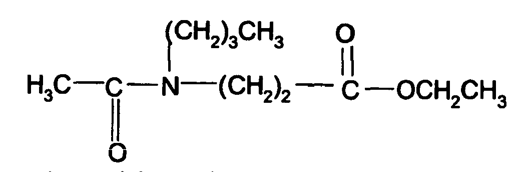 tel que le produit vendu sous la dénomination commerciale Repellent R3535 par la société MERCK ;
le N-lauroylsarcosinate d'isopropyle de formule : 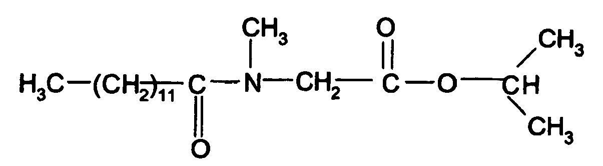 tel que le produit vendu sous le nom ELDEW SL-205 par la société Ajimoto.
le N,N-diéthyl-toluamide de formule : 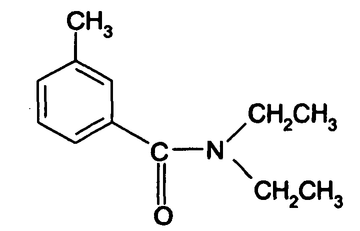 tel que le produit vendu sous le nom commercial DEET par la société Showa Denko.

L'huile ou les huiles présentant dans leur structure au moins une fonction amide telles que définies précédemment sont présentes dans les compositions selon l'invention à des concentrations allant de préférence de 0,1 à 40% en poids et plus préférentiellement de 1 à 20% en poids par rapport au poids total de la composition.

Les dérivés arylalkyl benzoates conformes à l'invention sont de préférence choisis parmi ceux de formule (2) ou (3) suivante : 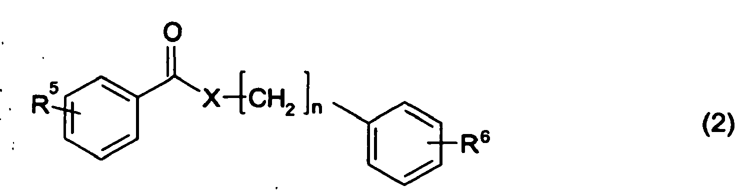 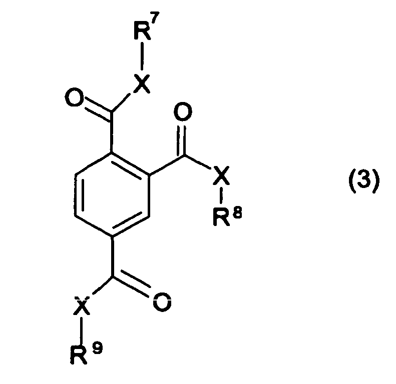 dans lesquelles :
X désigne O, S ou N ;
n est un entier de 1 à 10 et plus préférentiellement de 2 à 6 ;
R⁵ et R⁶, identiques ou différents, désignent un atome d'hydrogène ; le groupe hydroxyle ; un radical alcoxy en C₁-C₄ linéaire ou ramifié (de préférence méthoxy ou éthoxy) ; le radical nitro ; le radical amino ; le radical C₆H₆SO₂ ;
R⁷, R⁸ et R⁹, identiques ou différents, désignent un groupe de formule : 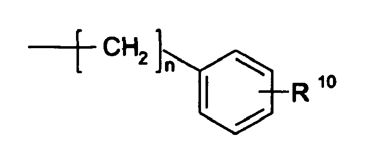
où n a la même signification indiquée ci-dessus ; R¹⁰ désigne un atome d'hydrogène ; le groupe hydroxyle ; un radical alcoxy en C₁-C₄ linéaire ou ramifié (de préférence méthoxy ou éthoxy) ; le radical nitro ; le radical amino ; le radical C₆H₆SO₂.

Les dérivés arylalkyl benzoates conformes à l'invention et leurs synthèses sont connus depuis longtemps dans la littérature chimique et notamment dans le brevet PL55230.

Parmi les dérivés arylalkyl benzoates mentionnés ci-dessus, on utilisera plus particulièrement le 2-phenylethyl benzoate de formule : 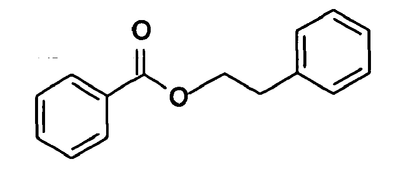 comme le produit commercial X-TEND 226 ® vendu par la société ISP.

Les dérivés arylalkyl benzoates conformes à l'invention peuvent être présents dans les compositions conformes à l'invention à des teneurs allant de 0,1 à 40% en poids et plus préférentiellement de 1 à 30 % en poids par rapport au poids total de la composition.

Le dérivé de 1,3,5-triazine répond à la formule (I) suivante : 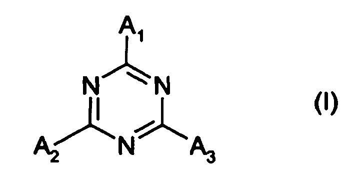 dans laquelle les radicaux A1, A2 et A3, identiques ou différents sont choisis parmi les groupes de formules (II) à (IX) suivantes : 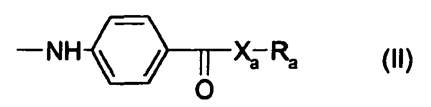 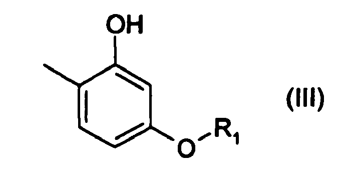 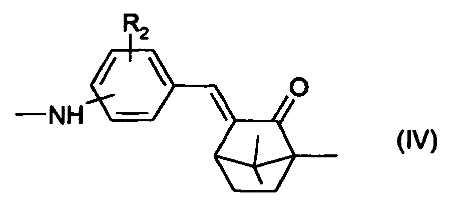 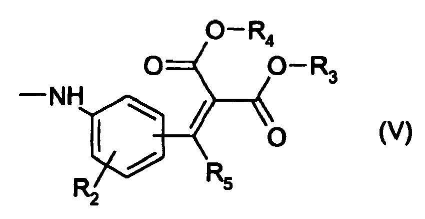 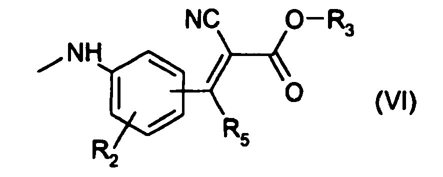 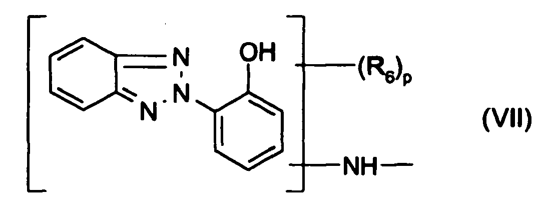 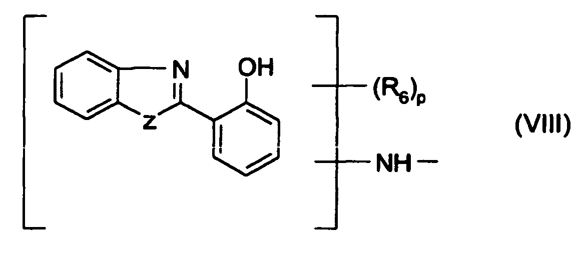 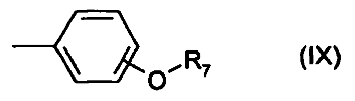 dans lesquelles :
- Xₐ (chacun des Xa peut être identique ou différent) représente l'oxygène ou -NH-;
- Rₐ (chacun des Rₐ peut être identique ou différent) est choisi parmi l'hydrogène; un métal alcalin; un radical ammonium éventuellement substitué par un ou plusieurs radicaux alkyles linéaire ou ramifié en C₁-C₁₈ ou hydroxyalkyles linéaire ou ramifié en C₁-C₁₈; un radical alkyle linéaire ou ramifié en C₁-C₁₈, de préférence en C₆-C₁₂; un radical cycloalkyle en C₅-C₁₂ éventuellement substitué par un ou plusieurs radicaux alkyles en C₁-C₄; un radical polyoxyéthyléné comprenant de 1 à 6 unités d'oxyde d'éthylène et dont le groupe OH terminal est méthylé ; un radical de formule (X), (XI) ou (XII) suivantes : 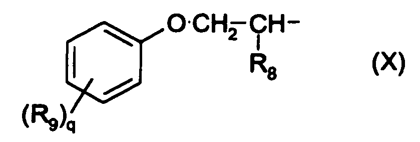 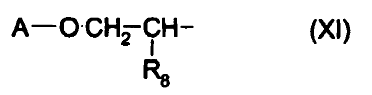 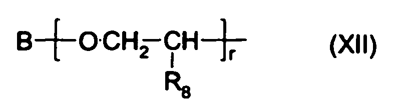 dans lesquelles :
   - R₈ est l'hydrogène ou un radical méthyle;
   - R₉ est un radical alkyle en C₁-C₉;
   - q est un nombre entier égal à 0; 1; 2; 3;
   - r est un nombre entier égal à 1; 2; 3; 4; 5; 6; 7; 8; 9; 10;
   - A est un radical alkyle en C₄-C₈ ou un radical cycloalkyle en C₅-C₈ ;
   - B est choisi parmi : un radical alkyle linéaire ou ramifié en C₁-C₈; un radical cycloalkyle en C₅-C₈; un radical aryle éventuellement substitué par un ou plusieurs radicaux alkyles en C₁-C₄.
   - R₁ désigne un radical alkyle en C₃-C₁₈ ; un radical alcényle en C₂-C₁₈ ; un reste de formule -CH₂-CH(OH)-CH₂-OT₁ où T₁ est un atome d'hydrogène ou un radical alkyle en C₁-C₈ ; un reste de formule (XIII) suivante : 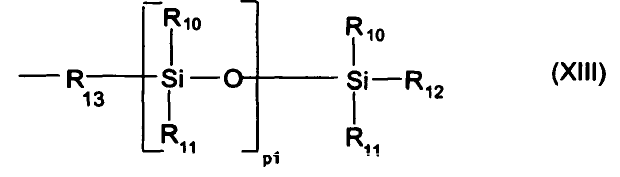
   dans laquelle :
   - R₁₃ désigne une liaison covalente ; un radical alkyle linéaire ou ramifié en C₁-C₄ ou bien un radical de formule -Cₘ₁H₂ₘ₁-O- où m₁ est un entier égal à 1; 2; 3; 4 ;
   - p₁ est un entier égal à 0; 1; 2; 3; 4; 5 ;
   - les radicaux R₁₀, R₁₁ et R₁₂, identiques ou différents, désignent un radical alkyle en C₁₋C₁₈ ; un radical alcoxy en C₁-C₁₈ ou un radical de formule : 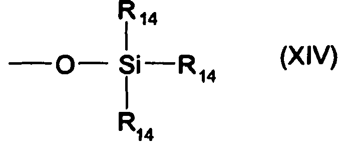 où R₁₄ est un radical alkyle en C₁-C₅.
   - R₂ désigne un atome d'hydrogène, un radical alkyle en C₁-C₄ linéaire ou ramifié ou un radical alkoxy en C₁-C₄ ;
   - R₃ et R₄ , identiques ou différents désignent un radical alkyle linéaire ou ramifié en C₁₋C₂₀ ;
   - R₅ représente un atome d'hydrogène ou un radical phényle éventuellement substitué par un halogène ou par un radical alkyle en C₁-_{C4} ou par un radical alcoxy en C₁-C₄ ;
   - R₆ est un radical alkyle en C₁-C₈ linéaire ou ramifié, alkoxy en C₁-C₃ étant entendu que, dans ce dernier cas, deux R₆ adjacents d'un même noyau aromatique peuvent former ensemble un groupement alkylidène dioxy dans lequel le groupe alkylidène contient de 1 à 2 atomes de carbone, OH, NHCOCH₃ ou NH₂,
   - R₇ désigne un atome d'hydrogène, un radical alkyle en C₁-C₁₀, un radical de formule : - (CH₂CHR₅-O)ₙ₁R₈ où n₁ est un nombre de 1 à 16, ou bien un radical de structure -CH₂-CH-(OH)-CH₂OT₁ avec R₈ et T₁ ayant la même signification indiquée ci-dessus. - Z représente l'oxygène, le soufre, -NH- ou -NR₃ - avec R₃ représentant un radical alkyle en C₁-C₂₀, linéaire ou ramifié; - p est 0, 1, 2 ou 3,

A₁ peut également être un halogène, un radical -N(R₃)₂, les deux R₃ pouvant former ensemble un cycle de 4 ou 5 atomes de carbone, ou un groupe -OR₃, R₃ ayant la même définition qu'au dessus.

Une première famille plus particulièrement préférée de dérivés de 1,3,5-triazine, notamment décrite dans le document EP-A-0 517 104, est celle des 1,3,5-triazines répondant à la formule (I) dans laquelle les A1, A2 et A3 sont de formule (II) et présentant l'ensemble des caractéristiques suivantes :
- l'un des groupements Xₐ-Rₐ représente le radical -NH-Rₐ avec Ra choisi parmi : un radical cycloalkyle en C₅-C₁₂ éventuellement substitué par un ou plusieurs radicaux alkyles en C₁-C₄; un radical de formule (X), (XI) ou (XII) ci-dessus dans lesquelles :

- B est un radical alkyle en C₁-C₄ ;
- R₉ est le radical méthyle ;
- les 2 autres Xₐ-Rₐ représentent le radical -O-Rₐ avec Rₐ, identiques ou différents choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄; un radical de formule (X), (XI) ou (XII) ci-dessus dans lesquelles :

- B est un radical alkyle en C₁-C₄;
- R₉ est le radical méthyle.

Une deuxième famille plus particulièrement préférée de dérivés de 1,3,5-triazine, notamment décrite dans le document EP-A-0 570 838, est celle des 1,3,5-triazines répondant à la formule (I) dans laquelle les A₁, A₂ et A₃ sont de formule (II) et présentant l'ensemble des caractéristiques suivantes :
- un ou deux Xₐ-Rₐ représente le radical -NH-Rₐ , avec Rₐ choisi parmi : un radical alkyle linéaire ou ramifié en C₁-C₁₈; un radical cycloalkyle en C₅-C₁₂ éventuellement substitué avec un ou plusieurs radicaux alkyles en C₁-C₄; un radical de formule (X), (XI) ou (XII) ci-dessus dans lesquelles B est un radical alkyle en C₁-C₄ et R₉ est le radical méthyle ;
- le ou les deux autres Xₐ-Rₐ étant le radical -O-Rₐ avec Rₐ, identiques ou différents choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄; un radical de formule (X), (XI) ou (XII) ci-dessus dans lesquelles B est un radical alkyle en C₁-C₄ et R₉ est le radical méthyle.

Une 1,3,5-triazine particulièrement préférée de cette deuxième famille est la 2-[(p-(tertiobutylamido)anilino]-4,6-bis-[(p-(2'-éthylhexyl-1'-oxycarbonyl)anilino]-1,3,5-triazine ou « Diethylhexyl Butamido Triazone » vendue sous le nom commercial « UVASORB HEB » par SIGMA 3V et répondant à la formule suivante : 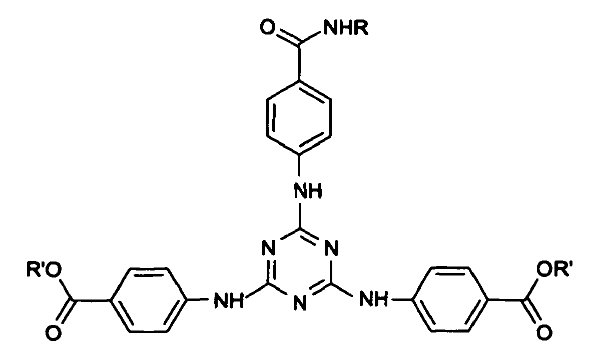 dans laquelle R' désigne un radical éthyl-2 hexyle et R désigne un radical tert-butyle.

Une troisième famille préférée de composés utilisables dans le cadre de la présente invention, et qui est notamment décrite dans le document US 4,724,137, est celle des 1,3,5-triazines répondant à la formule (1) dans laquelle les A₁, A₂ et A₃ sont de formule (II) et présentant l'ensemble des caractéristiques suivantes :
- Xₐ sont identiques et représentent l'oxygène;
- Rₐ, identiques ou différents et représentent un radical alkyle en C₆-C₁₂ ou un radical polyoxyéthyléné comprenant de 1 à 6 unités d'oxyde d'éthylène et dont le groupe OH terminal est méthylé.

Une 1,3,5-triazine particulièrement préférée de cette troisième famille est la 2,4,6-tris[p-(2'-éthylhexyl-1'-oxycarbonyl)anilino]-1,3,5-triazine ou « Ethylhexyl Triazone » vendue notamment sous la dénomination commerciale de "UVINUL T 150" par la Société BASF et répond à la formule suivante : 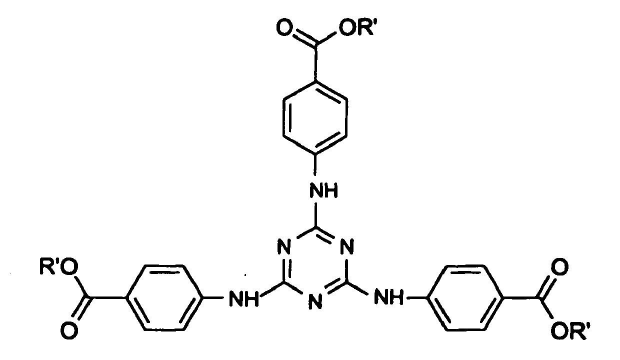 dans laquelle R' désigne un radical 2-éthyl hexyle.

Une quatrième famille préférée de composés utilisables dans le cadre de la présente invention, et qui est notamment décrite dans la demande de brevet EP-A-0775698 est celle des 1,3,5-triazines répondant à la formule (I) dans laquelle les A₁ et A₂ sont de formule (III) et A₃ est de formule (IX) et présentant l'ensemble des caractéristiques suivantes : R₁, identiques ou différents, désignent un radical alkyle en C₃-C₁₈ ; un radical alcényle en C₂-C₁₈ ou bien un reste de formule -CH₂-CH(OH)-CH₂-OT₁ où T₁ est un atome d'hydrogène ou un radical alkyle en C₁-C₈ ; R₇ désigne un atome d'hydrogène, un radical alkyle en C₁-C₁₀.

Une 1,3,5-triazine particulièrement préférée de cette quatrième famille est la 2,4-bis {[4-2-éthyl-hexyloxy)]-2-hydroxy]-phenyl}-6-(4-méthoxy-phenyl)-1,3,5-triazine ou « Anisotriazine » vendue sous le nom commercial «TINOSORB S » par CIBA SPECIALTY CHEMICALS et répond à la formule suivante : 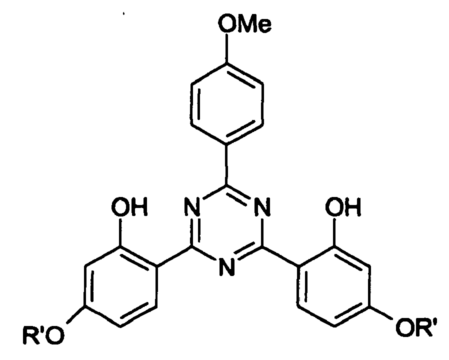 dans laquelle R' désigne un radical éthyl-2 hexyle.

Une cinquième famille préférée de composés utilisables dans le cadre de la présente invention, et qui est notamment décrite dans les demandes de brevet EP507691, EP507692, EP790243 et EP944624 et dont le contenu technique est intégré totalement à la présente description est celle des 1,3,5-triazines répondant à la formule (I) dans laquelle les A1, A2 et A3 sont de formules (VII) à (XI) citées précédemment.

A titre d'exemples de ces composés de formule utilisables, on peut citer :
- la 2,4,6-tris-(4'-amino benzalmalonate de diisobutyle)-s-triazine,
la 2,4,6-tris-(4'-amino benzalmalonate de di(2-éthylhexyle))-s-triazine,
la 2,4,6-tris-(4'-amino benzalmalonate de di(2-éthylhexyle))-6-chloro-s-triazine,
la 2,4,6-tris-(4'-amino benzalmalonate de di(2-éthylhexyle))-6-(4'-aminobenzoate de 2-éthylhexyle)-s-triazine,
la 2,4,6-tris-(4'-amino benzalmalonate de diisobutyle)-6-butoxy-s-triazine,
la 2,4,6-tris-(4'-amino benzalmalonate de diisobutyle)-6-(2-éthylhexylamino)-s-triazine,
la 2,4-bis-(4'-aminobenzylidène camphre)-6-(2-éthylhexyl-amino)-s-triazine,
la 2,4-bis-(4'-aminobenzylidène camphre)-6-(4'-amino benzalmalonate de diisobutyle)-s-triazine,
la 2,4,6-tris(4'-amino benzalmalonate de diéthyle)-s-triazine,
la 2,4,6-tris(4'-amino benzalmalonate de diisopropyle)-s-triazine,
la 2,4,6-tris(4'-amino benzalmalonate de diméthyle)-s-triazine,
la 2,4,6-tris(α-cyano-4-aminocinnamate d'éthyle)-s-triazine.
la 2,4,6-tris[(3'-benzotriazol-2-yl-2'-hydroxy-5'-methyl) phenylamino]-s-triazine,
2,4,6-tris[(3'-benzotriazol-2-yl-2'-hydroxy-5'-ter-octyl) phénylamino]-s-triazine.

Une 1,3,5-triazine particulièrement préférée de cette cinquième famille est la 2,4,6-tris-(4'-amino benzalmalonate de diisobutyle)-s- triazine qui répond à la formule suivante :

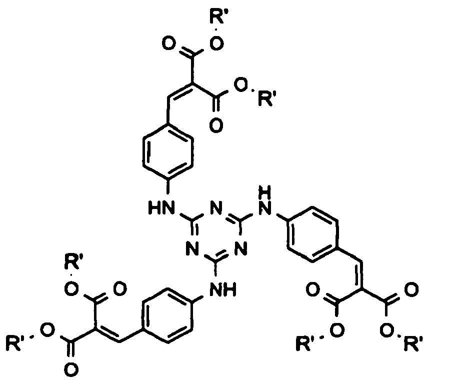

Les compositions selon la présente invention comprennent de préférence, dans un milieu physiologiquement acceptable de 0,05 à 15%, de préférence de 0,1 à 10 % de dérivés 1,3,5 triazine en poids par rapport au poids total de ladite composition.

Ladite composition selon la présente invention est de préférence une composition cosmétique contenant outre le dérivé 1,3,5 triazine en tant que filtre organique, au moins un autre filtre organique et/ou au moins un autre filtre minéral complémentaire hydrosoluble, liposoluble ou insoluble dans les solvants cosmétiques couramment utilisés.

Les compositions conformes à l'invention peuvent comporter en plus d'autres agents photoprotecteurs organiques ou inorganiques complémentaires actifs dans l'UVA et/ou l'UVB hydrosolubles ou liposolubles ou bien insolubles dans les solvants cosmétiques couramment utilisés.

Les agents photoprotecteurs organiques complémentaires sont notamment choisis parmi les anthranilates ; les dérivés cinnamiques ; les dérivés salicyliques, les dérivés du camphre ; les dérivés de la benzophénone ; les dérivés de β,β-diphénylacrylate; les dérivés de benzotriazole ; les dérivés de benzalmalonate ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans s les demandes US5,237,071, US 5,166,355, GB2303549, DE 197 26 184 et EP893119 ; les dérivés de benzoxazole tels que décrits dans les demandes de brevet EP0832642, EP1027883, EP1300137 et DE10162844 ; les polymères filtres et silicones filtres tels que ceux décrits notamment dans la demande WO-93/04665 ; les dimères dérivés d'α-alkylstyrène tels que ceux décrits dans la demande de brevet DE19855649 ; les 4,4-diarylbutadiènes tels que décrits dans les demandes EP0967200, DE19746654, DE19755649, EP-A-1008586, EP1133980 et EP133981 et leurs mélanges.

Comme exemples de filtres organiques complémentaires, on peut citer ceux désignés ci-dessous sous leur nom INCI :

### Dérivés de l'acide para-aminobenzoique :

PABA,
Ethyl PABA,
Ethyl Dihydroxypropyl PABA,
Ethylhexyl Diméthyl PABA vendu notamment sous le nom « ESCALOL 507 » par ISP,
Glyceryl PABA,
PEG-25 PABA vendu sous le nom « UVINUL P25 » par BASF,

### Dérivés salicyliques :

Homosalate vendu sous le nom « Eusolex HMS » par Rona/EM Industries,
Ethylhexyl Salicylate vendu sous le nom « NEO HELIOPAN OS » par Haarmann et REIMER,
Dipropyleneglycol Salicylate vendu sous le nom « DIPSAL » par SCHER,
TEA Salicylate, vendu sous le nom « NEO HELIOPAN TS » par Haarmann et REIMER,

### Dérivés cinnamiques :

Ethylhexyl Methoxycinnamate vendu notamment sous le nom commercial « PARSOL MCX » par HOFFMANN LA ROCHE,
Isopropyl Methoxy cinnamate,
Isoamyl Methoxy cinnamate vendu sous le nom commercial « NEO HELIOPAN E 1000 » par HAARMANN et REIMER,
Cinoxate,
DEA Methoxycinnamate,
   - Diisopropyl Methylcinnamate,
Glyceryl Ethylhexanoate Dimethoxycinnamate

### Dérivés de β,β-diphénylacrylate :

Octocrylene vendu notamment sous le nom commercial « UVINUL N539 » par BASF,
Etocrylene, vendu notamment sous le nom commercial « UVINUL N35 » par BASF,

### Dérivés de la benzophénone :

Benzophenone-1 vendu sous le nom commercial « UVINUL 400 » par BASF,
Benzophenone-2 vendu sous le nom commercial « UVINUL D50 » par BASF
Benzophenone-3 ou Oxybenzone, vendu sous le nom commercial « UVINUL M40 » par BASF,
Benzophenone-4 vendu sous le nom commercial « UVINUL MS40 » par BASF,
Benzophenone-5
Benzophenone-6 vendu sous le nom commercial « Helisorb 11 » par Norquay
Benzophenone-8 vendu sous le nom commercial « Spectra-Sorb UV-24 » par American Cyanamid
Benzophenone-9 vendu sous le nom commercial« UVINUL DS-49» par BASF,
Benzophenone-12
2-(4-diethylamino-2-hydroxybenzoyl)-benzoate de n-hexyle.

### Dérivés du benzylidène camphre :

3-Benzylidene camphor fabriqué sous le nom « MEXORYL SD» par CHIMEX,
4-Methylbenzylidene camphor vendu sous le nom « EUSOLEX 6300 » par MERCK ,
Benzylidene Camphor Sulfonic Acid fabriqué sous le nom « MEXORYL SL» par CHIMEX,
Camphor Benzalkonium Methosulfate fabriqué sous le nom « MEXORYL SO » par CHIMEX,
Terephthalylidene Dicamphor Sulfonic Acid fabriqué sous le nom « MEXORYL SX » par CHIMEX,
Polyacrylamidomethyl Benzylidene Camphor fabriqué sous le nom « MEXORYL SW » par CHIMEX,

### Dérivés du phenyl benzimidazole :

Phenylbenzimidazole Sulfonic Acid vendu notamment sous le nom commercial « EUSOLEX 232 » par MERCK,
Disodium Phenyl Dibenzimidazole Tetra-sulfonate vendu sous le nom commercial commercial « NEO HELIOPAN AP » par Haarmann et REIMER,

### Dérivés du phenyl benzotriazole :

Drometrizole Trisiloxane vendu sous le nom « Silatrizole » par RHODIA CHIMIE ,
Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,

### Dérivés anthraniliques :

Menthyl anthranilate vendu sous le nom commercial commercial « NEO HELIOPAN MA » par Haarmann et REIMER,

### Dérivés d'imidazolines :

Ethylhexyl Dimethoxybenzylidene Dioxoimidazoline Propionate,

### Dérivés du benzalmalonate :

Polyorganosiloxane à fonctions benzalmalonate comme le Polysilicone-15 vendu sous la dénomination commerciale « PARSOL SLX » par HOFFMANN LA ROCHE

### Dérivés de 4,4-diarylbutadiène :

- 1,1-dicarboxy (2,2'-diméthyl-propyl)-4,4-diphénylbutadiène

### Dérivés de benzoxazole :

2,4-bis-[5-1 (diméthylpropyl)benzoxazol-2-yl-(4-phenyl)-imino]-6-(2-ethylhexyl)-imino-1,3,5-triazine vendu sous le nom d'Uvasorb K2A par Sigma 3V
et leurs mélanges.

Les filtres UV organiques complémentaires préférentiels sont choisis parmi Homosalate
Ethylhexyl Salicylate,
Ethylhexyl Methoxycinnamate
Octocrylene,
Phenylbenzimidazole Sulfonic Acid,
Benzophenone-3,
Benzophenone-4,
Benzophenone-5,
2-(4-diethylamino-2-hydroxybenzoyl)-benzoate de n-hexyle.
4-Methylbenzylidene camphor,
Terephthalylidene Dicamphor Sulfonic Acid,
Disodium Phenyl Dibenzimidazole Tetra-sulfonate,
Methylène bis-Benzotriazolyl Tetramethylbutylphénol
Drometrizole Trisiloxane
Polysilicone-15
1,1-dicarboxy (2,2'-diméthyl-propyl)-4,4-diphénylbutadiène
2,4-bis-[5-1 (diméthylpropyl)benzoxazol-2-yl-(4-phenyl)-imino]-6-(2-ethykhexyl)-imino-1,3,5-triazine
et leurs mélanges.

Les agents photoprotecteurs complémentaires inorganiques sont choisis parmi des pigments et plus préférentiellement encore des nanopigments (taille moyenne des particules primaires : généralement entre 5 nm et 100 nm, de préférence entre 10 nm et 50 nm) d'oxydes métalliques traités ou non comme par exemple des nanopigments d'oxyde de titane (amorphe ou cristallisé sous forme rutile et/ou anatase), de fer, de zinc, de zirconium ou de cérium.

Les nanopigments traités sont des pigments qui ont subi un ou plusieurs traitements de surface de nature chimique, électronique, mécanochimique et/ou mécanique avec des composés tels que décrits par exemple dans Cosmetics & Toiletries, Février 1990, Vol. 105, p 53-64, tels que des aminoacides, de la cire d'abeille, des acides gras, des alcools gras, des tensio-actifs anioniques, des lécithines, des sels de sodium, potassium, zinc, fer, ou aluminium d'acides gras, des alcoxydes métalliques (de titane ou d'aluminium), du polyéthylène, des silicones, des protéines (collagène, élastine) des alcanolamines, des oxydes de silicium, des oxydes métalliques, de l'hexamétaphosphate de sodium, de l'alumine ou de la glycérine.

Les nanopigments traités peuvent être plus particulièrement des oxydes de titane traités par:
- la silice et l'alumine tels que les produits « Microtitanium Dioxide MT 500 SA » et « Microtitanium Dioxide MT 100 SA » de la société TAYCA, et les produits « Tioveil Fin », « Tioveil OP », « Tioveil MOTG » et « Tioveil IPM » de la société TIOXIDE,
- l'alumine et le stéarate d'aluminium tels que le produit « Microtitanium Dioxide MT 100 T » de la société TAYCA,
- l'alumine et le laurate d'aluminium tels que le produit « Microtitanium Dioxide MT 100 S » de la société TAYCA,
- des oxydes de fer et le stéarate de fer tels que le produit « Microtitanium Dioxide MT 100 F » de la société TAYCA,
- la silice, l'alumine et la silicone tels que les produits « Microtitanium Dioxide MT 100 SAS », « Microtitanium Dioxide MT 600 SAS » et « Microtitanium Dioxide MT 500 SAS » de la société TAYCA,
- l'hexamétaphosphate de sodium tels que le produit « Microtitanium Dioxide MT 150 W » de la société TAYCA,
- l'octyltriméthoxysilane tels que le produit « T-805 » de la société DEGUSSA,
- l'alumine et l'acide stéarique tels que le produit « UVT-M160 » de la société KEMIRA,
- l'alumine et la glycérine tels que le produit « UVT-M212» de la société KEMIRA,
- l'alumine et la silicone tels que le produit « UVT-M262» de la société KEMIRA.

D'autres nanopigments d'oxyde de titane traités avec une silicone sont de préférence le TiO₂ traité par l'octyl triméthyl silane et dont la taille moyenne des particules élémentaires est comprise entre 25 et 40 nm tel que celui vendu sous la dénomination commerciale "T 805" par la société DEGUSSA SILICES, le TiO2 traité par un polydiméthylsiloxane et dont la taille moyenne des particules élémentaires est de 21 nm tel que celui vendu sous la dénomination commerciale "70250 CARDRE UF TiO2SI3" par la société CARDRE, le TiO2 anatase/rutile traité par un polydiméthylhydrogénosiloxane et dont la taille moyenne des particules élémentaires est de 25 nm tel que celui vendu sous la dénomination commerciale "MICRO TITANIUM DIOXYDE USP GRADE HYDROPHOBIC" par la société COLOR TECHNIQUES.

Les nanopigments d'oxyde de titane non enrobés sont par exemple vendus par la société TAYCA sous les dénominations commerciales "MICROTITANIUM DIOXIDE MT 500 B" ou "MICROTITANIUM DIOXIDE MT600 B", par la société DEGUSSA sous la dénomination "P 25", par la société WACKHER sous la dénomination "Oxyde de titane transparent PW", par la société MIYOSHI KASEI sous la dénomination "UFTR", par la société TOMEN sous la dénomination "ITS" et par la société TIOXIDE sous la dénomination "TIOVEIL AQ".

Les nanopigments d'oxyde de zinc non enrobés, sont par exemple
- ceux commercialisés sous la dénomination "Z-COTE" par la société SUNSMART ;
- ceux commercialisés sous la dénomination "NANOX" par la société ELEMENTIS ;
- ceux commercialisés sous la dénomination "NANOGARD WCD 2025" par la société NANOPHASE TECHNOLOGIES ;

Les nanopigments d'oxyde de zinc enrobés sont par exemple
- ceux commercialisés sous la dénomination "OXIDE ZINC CS-5" par la société Toshibi (ZnO enrobé par polymethylhydrogenesiloxane) ;
- ceux commercialisés sous la dénomination "NANOGARD ZINC OXIDE FN" par la société NANOPHASE TECHNOLOGIES (en dispersion à 40% dans le Finsolv TN, benzoate d'alcools en C₁₂-C₁₅) ;
- ceux commercialisés sous la dénomination "DAITOPERSION ZN-30" et "DAITOPERSION ZN-50" par la société Daito (dispersions dans cyclopolyméthylsiloxane /polydiméthylsiloxane oxyéthyléné, contenant 30% ou 50% de nano-oxydes de zinc enrobés par la silice et le polyméthylhydrogènesiloxane) ;
- ceux commercialisés sous la dénomination "NFD ULTRAFINE ZNO" par la société Daikin (ZnO enrobé par phosphate de perfluoroalkyle et copolymère à base de perfluoroalkyléthyle en dispersion dans du cyclopentasiloxane) ;
- ceux commercialisés sous la dénomination "SPD-Z1" par la société Shin-Etsu (ZnO enrobé par polymère acrylique greffé silicone, dispersé dans cyclodiméthylsiloxane) ;
- ceux commercialisés sous la dénomination "ESCALOL Z100" par la société ISP (ZnO traité alumine et dispersé dans le mélange methoxycinnamate d'ethylhexyle / copolymère PVP-hexadecene / methicone) ;
- ceux commercialisés sous la dénomination "FUJI ZNO-SMS-10" par la société Fuji Pigment (ZnO enrobé silice et polymethylsilsesquioxane) ;
- ceux commercialisés sous la dénomination "NANOX GEL TN" par la société Elementis (ZnO dispersé à 55% dans du benzoate d'alcools en C₁₂-C₁₅ avec polycondensat d'acide hydroxystéarique).

Les nanopigments d'oxyde de cérium non enrobé est vendu sous la dénomination "COLLOIDAL CERIUM OXIDE" par la société RHONE POULENC.
Les nanopigments d'oxyde de fer non enrobés sont par exemple vendus par la société ARNAUD sous les dénominations "NANOGARD WCD 2002 (FE 45B)", "NANOGARD IRON FE 45 BL AQ", "NANOGARD FE 45R AQ, "NANOGARD WCD 2006 (FE 45R)", ou par la société MITSUBISHI sous la dénomination "TY-220".

Les nanopigments d'oxyde de fer enrobés sont par exemple vendus par la société ARNAUD sous les dénominations "NANOGARD WCD 2008 (FE 45B FN)", "NANOGARD WCD 2009 (FE 45B 556)", "NANOGARD FE 45 BL 345", "NANOGARD FE 45 BL", ou par la société BASF sous la dénomination "OXYDE DE FER TRANSPARENT".

On peut également citer les mélanges d'oxydes métalliques, notamment de dioxyde de titane et de dioxyde de cérium, dont le mélange équipondéral de dioxyde de titane et de dioxyde de cérium enrobés de silice, vendu par la société IKEDA sous la dénomination "SUNVEIL A", ainsi que le mélange de dioxyde de titane et de dioxyde de zinc enrobé d'alumine, de silice et de silicone tel que le produit "M 261" vendu par la société KEMIRA ou enrobé d'alumine, de silice et de glycérine tel que le produit "M 211" vendu par la société KEMIRA.

Les nanopigments peuvent être introduits dans les compositions selon l'invention tels quels ou sous forme de pâte pigmentaire, c'est-à-dire en mélange avec un dispersant, comme décrit par exemple dans le document GB-A-2206339.

Les agents photoprotecteurs additionnels sont généralement présents dans les compositions selon l'invention dans des proportions allant de 0,01 à 20% en poids par rapport au poids total de la composition, et de préférence allant de 0,1 à 10% en poids par rapport au poids total de la composition.

Les compositions selon l'invention peuvent également contenir des agents de bronzage et/ou de brunissage artificiels de la peau (agents autobronzants), et plus particulièrement la dihydroxyacétone (DHA). Ils sont présents de préférence dans des quantité allant 0,1 à 10% en poids par rapport au poids total de la composition.

Les compositions conformes à la présente invention peuvent comprendre en outre des adjuvants cosmétiques classiques notamment choisis parmi les corps gras, les solvants organiques, les épaississants ioniques ou non ioniques, hydrophiles ou lipophiles, les adoucissants, les humectants, les opacifiants, les stabilisants, les émollients, les silicones, les agents anti-mousse, les parfums, les conservateurs, les tensioactifs anioniques, cationiques, non-ioniques, zwitterioniques ou amphotères, des actifs, les charges, les polymères, les propulseurs, les agents alcalinisants ou acidifiants ou tout autre ingrédient habituellement utilisé dans le domaine cosmétique et/ou dermatologique.

Les corps gras peuvent être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (paraffine); végétales (huile d'amande douce, de macadamia, de pépin de cassis, de jojoba) ; synthétiques comme le perhydrosqualène, les alcools, les acides ou les esters gras (comme le benzoate d'alcools en C₁₂-C₁₅ vendu sous la dénomination commerciale « Finsolv TN » par la société WITCO, le palmitate d'octyle, le lanolate d'isopropyle, les triglycérides dont ceux des acides caprique/caprylique), les esters et éthers gras oxyéthylénés ou oxypropylénés; les huiles siliconées (cyclométhicone, polydiméthysiloxanes ou PDMS) ou fluorées, les polyalkylènes.

Comme composés cireux, on peut citer la paraffine, la cire de carnauba, la cire d'abeille, l'huile de ricin hydrogénée.

Parmi les solvants organiques, on peut citer les alcools et polyols inférieurs. Ces derniers peuvent être choisis parmi les glycols et les éthers de glycol comme l'éthylène glycol, le propylène glycol, le butylène glycol, le dipropylène glycol ou le diéthylène glycol.

Comme épaississants hydrophiles, on peut citer les polymères carboxyvinyliques tels que les carbopols (carbomers) et les Pemulen (Copolymère acrylate/C10-C30-alkylacrylate) ; les polyacrylamides comme par exemple les copolymères réticulés vendus sous les noms Sepigel 305 (nom C.T.F.A. : polyacrylamide/C13-14 isoparaffin/Laureth 7) ou Simulgel 600 (nom C.T.F.A. : acrylamide / sodium acryloyldimethyltaurate copolymer /isohexadecane / polysorbate 80) par la société Seppic ; les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société Hoechst sous la dénomination commerciale « Hostacerin AMPS » (nom CTFA : ammonium polyacryldimethyltauramide) ; les dérivés cellulosiques tels que l'hydroxyéthylcellulose ; les polysaccharides et notamment les gommes telles que la gomme de xanthane ; et leurs mélanges.

Comme épaississants lipophiles, on peut citer les argiles modifiées telles que le l'hectorite et ses dérivés, comme les produits commercialisés sous les noms de Bentone.

Parmi les actifs, on peut citer :
- les agents anti-pollution et/ou agent anti-radicalaire ;
- les agents dépigmentants et/ou des agents pro-pigmentants ;
- les agents anti-glycation ;
- les inhibiteurs de NO-synthase ;
- les agents stimulant la synthèse de macromolécules dermiques ou épidermiques et/ou empêchant leur dégradation ;
- les agents stimulant la prolifération des fibroblastes ;
- les agents stimulant la prolifération des kératinocytes ;
- les agents myorelaxants ;
- les agents tenseurs.
- les agents desquamants ;
- les agents hydratants ;
- les agents anti-inflammatoires ;
- les agents agissant sur le métabolisme énergétique des cellules.
- les agents répulsifs contre les insectes
- les antagonistes de substances P ou de CRGP.

Bien entendu, l'homme de l'art veillera à choisir le ou les éventuels composés complémentaires cités ci-dessus et/ou leurs quantités de manière telle que les propriétés avantageuses attachées intrinsèquement aux compositions conformes à l'invention ne soient pas, ou substantiellement pas, altérées par la ou les adjonctions envisagées.

Les compositions selon l'invention peuvent être préparées selon les techniques bien connues de l'homme de l'art, en particulier celles destinées à la préparation d'émulsions de type huile-dans-eau ou eau-dans-huile. Elles peuvent se présenter en particulier sous forme d'émulsion, simple ou complexe (H/E, E/H, H/E/H ou E/H/E) telle qu'une crème, un lait, ou sous la forme d'un gel ou d'un gel crème, sous la forme d'une lotion, de poudre, de bâtonnet solide et éventuellement être conditionnée en aérosol et se présenter sous forme de mousse ou de spray.

De préférence, les compositions selon l'invention se présentent sous la forme d'une émulsion huile-dans-eau ou eau-dans huile.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant l'émulsion à obtenir (E/H ou H/E).

Comme tensioactifs émulsionnants utilisables pour la préparation des émulsions E/H, on peut citer par exemple les alkyl esters ou éthers de sorbitane, de glycérol ou de sucres ; les tensioactifs siliconés comme les dimethicone copolyols tels que le mélange de cyclomethicone et de dimethicone copolyol, vendu sous la dénomination « DC 5225 C » par la société Dow Corning, et les alkyl-dimethicone copolyols tels que le Laurylmethicone copolyol vendu sous la dénomination "Dow Corning 5200 Formulation Aid" par la société Dow Corning ; le Cetyl dimethicone copolyol tel que le produit vendu sous la dénomination Abil EM 90R par la société Goldschmidt et le mélange de cétyl diméthicone copolyol, d'isostearate de polyglycérole (4 moles) et de laurate d'hexyle vendu sous la dénomination ABIL WE 09 par la société Goldschmidt. On peut y ajouter aussi un ou plusieurs co-émulsionnants, qui, de manière avantageuse, peuvent être choisis dans le groupe comprenant les esters alkylés de polyol. Comme esters alkylés de polyol, on peut citer notamment les esters de glycérol et/ou de sorbitan et par exemple l'isostéarate de polyglycérol, tel que le produit commercialisé sous la dénomination Isolan Gl 34 par la société Goldschmidt, l'isostéarate de sorbitan, tel que le produit commercialisé sous la dénomination Arlacel 987 par la société ICI, l'isostéarate de sorbitan et le glycérol, tel que le produit commercialisé sous la dénomination Arlacel 986 par la société ICI, et leurs mélanges.

Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) ; les éthers d'alcools gras oxyalkylénés (oxyéthylénés et/ou oxypropylénés) ; les esters de sucres comme le stéarate de sucrose ; les éthers d'alcool gras et de sucre, notamment les alkylpolyglucosides (APG) tels que le décylglucoside et le laurylglucoside commercialisés par exemple par la société Henkel sous les dénominations respectives Plantaren 2000 et Plantaren 1200, le cétostéarylglucoside éventuellement en mélange avec l'alcool cétostéarylique, commercialisé par exemple sous la dénomination Montanov 68 par la société Seppic, sous la dénomination Tegocare CG90 par la société Goldschmidt et sous la dénomination Emulgade KE3302 par la société Henkel, ainsi que l'arachidyl glucoside, par exemple sous la forme du mélange d'alcools arachidique et béhénique et d'arachidylglucoside commercialisé sous la dénomination Montanov 202 par la société Seppic. Selon un mode particulier de réalisation de l'invention, le mélange de l'alkylpolyglucoside tel que défini ci-dessus avec l'alcool gras correspondant peut être sous forme d'une composition autoémulsionnante, comme décrit par exemple dans le document WO-A-92/06778.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

Les compositions selon l'invention trouvent leur application dans un grand nombre de traitements, notamment cosmétiques, de la peau, des lèvres et des cheveux, y compris le cuir chevelu, notamment pour la protection et/ou le soin de la peau, des lèvres et/ou des cheveux, et/ou pour le maquillage de la peau et/ou des lèvres.

Un autre objet de la présente invention est constitué par l'utilisation des compositions selon l'invention telles que ci-dessus définies pour la fabrication de produits pour le traitement cosmétique de la peau, des lèvres, des ongles, des cheveux, des cils, sourcils et/ou du cuir chevelu, notamment des produits de soin et des produits de maquillage.

Les compositions cosmétiques selon l'invention peuvent par exemple être utilisées comme produit de soin et/ou de protection solaire pour le visage et/ou le corps de consistance liquide à semi-liquide, telles que des laits, des crèmes plus ou moins onctueuses, gel-crèmes, des pâtes. Elles peuvent éventuellement être conditionnées en aérosol et se présenter sous forme de mousse ou de spray.

Les compositions selon l'invention sous forme de lotions fluides vaporisables conformes à l'invention sont appliquées sur la peau ou les cheveux sous forme de fines particules au moyen de dispositifs de pressurisation. Les dispositifs conformes à l'invention sont bien connus de l'homme de l'art et comprennent les pompes non-aérosols ou "atomiseurs", les récipients aérosols comprenant un propulseur ainsi que les pompes aérosols utilisant l'air comprimé comme propulseur. Ces derniers sont décrits dans les brevets US 4,077,441 et US 4,850,517 (faisant partie intégrante du contenu de la description).

Les compositions conditionnées en aérosol conformes à l'invention contiennent en général des agents propulseurs conventionnels tels que par exemple les composés hydrofluorés le dichlorodifluorométhane, le difluoroéthane, le diméthyléther, l'isobutane, le n-butane, le propane, le trichlorofluorométhane. Ils sont présents de préférence dans des quantités allant de 15 à 50% en poids par rapport au poids total de la composition.

Les exemples concrets, mais nullement limitatifs, illustrant l'invention, vont maintenant être donnés. On a réalisé les formulations solaires suivantes ; les quantités sont indiquées en pourcentages en poids :

| **Composition** | **Exemple 1** | **Exemple 2** |
|---|---|---|
| **PHASE A** | | |
| Polydiméthylsiloxane | 0,5 | 0,5 |
| Conservateurs | 1,0 | 1,0 |
| Acide stéarique | 1,5 | 1,5 |
| Mélange monostéarate de glycéryle/stéarate-PEG (100 OE) | 1,0 | 1,0 |
| Mélange cetylstéarylglucoside/alcool cetylstéarylique | 2,0 | 2,0 |
| Alcool cétylique | 0,5 | 0,5 |
| Butyl Methoxy Dibenzoylmethane | 2,0 | 2,0 |
| 2-phenylethyl benzoate (X-TEND 226 de ISP) | 10 | 10 |
| N-lauroyl isopropyl sarcosinate (ELDEW SL-205- Ajimoto) | 10 | 10 |
| Bis-Ethylhexyloxyphenol Methoxyphenyl Triazine (TINOSORB S par CIBA GEIGY) | 5 | - |
| Ethylhexyl Triazone (UVINUL T150 par BASF) | - | 5 |

| **PHASE B** | | |
|---|---|---|
| Eau désionisée | qsp 100 | qsp 100 |
| Sequestrant | 0,1 | 0,1 |
| Glycérine | 5,0 | 5,0 |
| Gomme de Xanthane | 0,2 | 0,2 |
| Phosphate de monocétyle | 1,0 | 1,0 |

| **PHASE C** | | |
|---|---|---|
| Isohexadécane | 1,0 | 1,0 |
| Copolymère acide acrylique/méthacrylate de stéaryle | 0,2 | 0,2 |
| Triéthanolamine | qs | qs |

On chauffe la phase aqueuse (Phase B) contenant l'ensemble de ses ingrédients à 80°C au bain marie. On chauffe la phase grasse (Phase A) contenant l'ensemble de ses ingrédients à 80°C au bain marie. On émulsionne A dans B sous agitation de type rotor-stator (appareil de la société Moritz). On incorpore la phase C et on laisse revenir à température ambiante sous agitation modérée. On introduit la triéthanolamine de façon à ajuster le pH à la valeur désirée en fin de fabrication.

## Revendications

1. Composition cosmétique ou dermatologique photoprotectrice, **caractérisée par le fait qu'**elle comprend :
a) au moins un dérivé de 1,3,5-triazine et
b) un mélange comprenant
(i) au moins une huile présentant dans sa structure au moins un motif amide et
(ii) au moins un dérivé arylalkyl benzoate.

2. Composition selon la revendication 1, où l'huile présentant dans sa structure au moins un motif amide est choisie parmi les composés de formule (1) suivante : 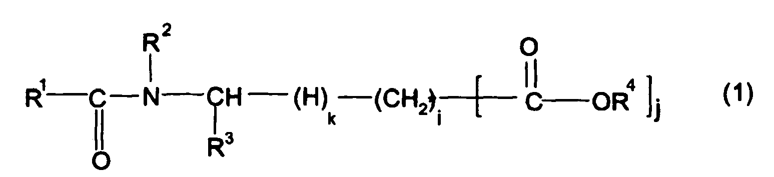 dans laquelle :
- le radical R¹ représente un radical hydrocarboné monovalent, saturé ou insaturé, aliphatique, cycloaliphatique ou cyclique, éventuellement fonctionnalisé, contenant de 1 à 30 atomes de carbone, de préférence de 1 à 22 atomes de carbone, bornes incluses ;
- les radicaux R², R³ et R⁴ qui peuvent être identiques ou différents, représentent l'hydrogène ou des radicaux hydrocarbonés monovalents, saturés ou insaturés, aliphatiques, cycloaliphatiques ou cycliques, éventuellement fonctionnalisés, contenant de 1 à 30 atomes de carbone, de préférence de 1 à 22 atomes de carbone, bornes incluses;
- k vaut 0 ou 1 ;
- i est un entier de 0 à 2 ;
- j vaut 0 ou 1 ;
- sous réserve que :
- lorsque j = 1 alors k vaut 0 et lorsque j = 0 alors i =0 et k =1.

3. Composition selon la revendication 2, où l'huile présentant dans sa structure au moins un motif amide de formule (1) est choisie parmi celles où :
R¹ représente un radical alkyle en C₁-C₂₂, linéaire ou ramifiée ; un radical alcényle en C₂-C₂₂, linéaire ou ramifié ; un radical aryle ;
R² représente un atome d'hydrogène ou un groupe alkyle en C₁-C₆, linéaire ou ramifiée ;
R³ représente un atome d'hydrogène ou un groupe alkyle en C₁-C₆, linéaire ou ramifiée ;
R⁴ représente un radical alkyle en C₁-C₁₀ linéaire ou ramifié, ou un radical alcényle en C₂-C₁₀ linéaire ou ramifié ou un reste stérol.

4. Composition selon la revendication 3, où l'huile présentant dans sa structure au moins un motif amide de formule (1) est choisie parmi :
le N-acetyl N-butylaminopropionate de formule suivante : 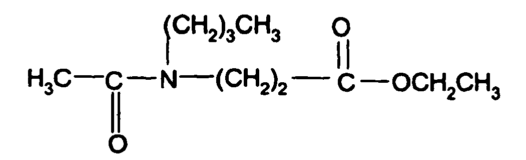
le N-lauroylsarcosinate d'isopropyle de formule : 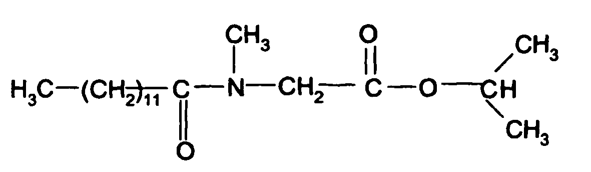
le N,N-diéthyl-toluamide de formule :

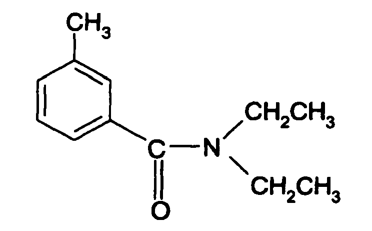

5. Composition selon l'une quelconque des revendications 1 à 4, où l'huile ou les huiles présentant dans leur structure au moins une fonction amide sont présentes à des concentrations allant de préférence de 0,1 à 40% en poids et plus préférentiellement de 1 à 20% en poids par rapport au poids total de la composition.

6. Composition selon l'une quelconque des revendications 1 à 5, où le dérivé arylalkyl benzoate est choisi parmi ceux de formule (2) ou (3) suivante : 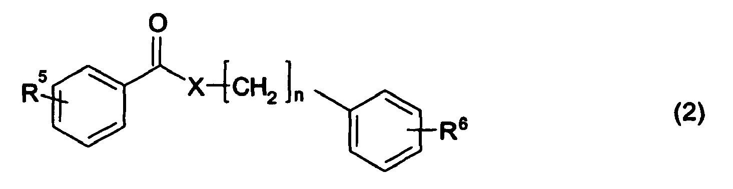 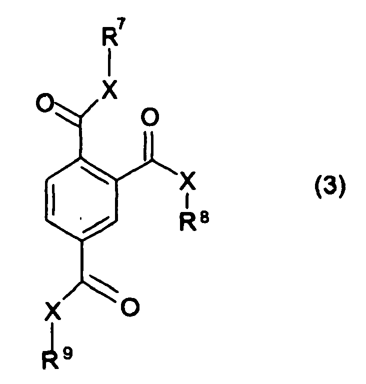 dans lesquelles :
X désigne O, S ou N ;
n est un entier de 1 à 10 et plus préférentiellement de 2 à 6 ;
R⁵ et R⁶, identiques ou différents, désignent un atome d'hydrogène ; le groupe hydroxyle ; un radical alcoxy en C₁-C₄ linéaire ou ramifié ; le radical nitro ; le radical amino ; le radical C₆H₆SO₂ ;
R⁷, R⁸ et R⁹, identiques ou différents, désignent un groupe de formule : 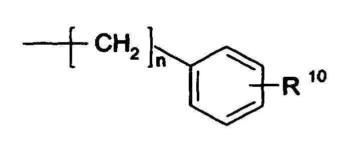
où n a la même signification indiquée ci-dessus ; R¹⁰ désigne un atome d'hydrogène ; le groupe hydroxyle ; un radical alcoxy en C₁-C₄ linéaire ou ramifié ; le radical nitro ; le radical amino ; le radical C₆H₆SO₂.

7. Composition selon la revendication 6, où le dérivé arylalkyl benzoate est le 2-phenylethyl benzoate de formule :

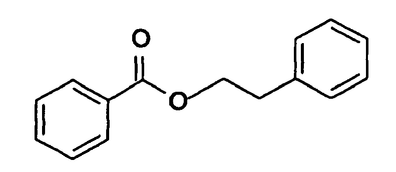

8. Composition selon l'une quelconque des revendications 1 à 7, où le composé benzoate d'alkylaryle est présent à des concentrations allant de 0,1 à 40% en poids et plus préférentiellement de 1 à 30 % en poids par rapport au poids total de la composition.

9. Composition selon l'une quelconque des revendications 1 à 8, où le dérivé de 1,3,5-triazine répond à la formule (I) suivante : 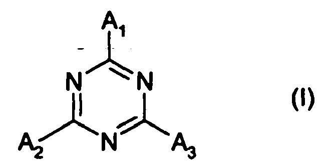 dans laquelle les radicaux A₁, A₂ et A₃, identiques ou différents sont choisis parmi les groupes de formules (II) à (IX) suivantes : 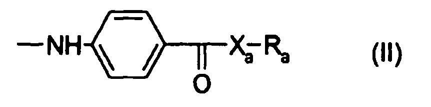 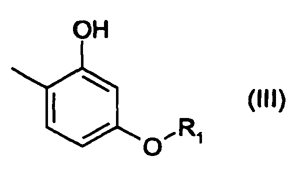 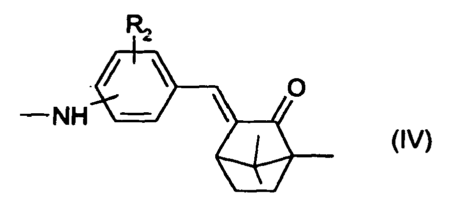 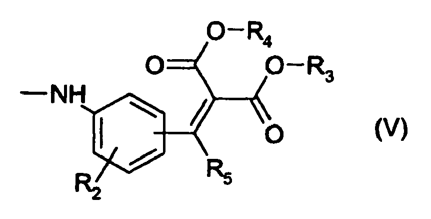 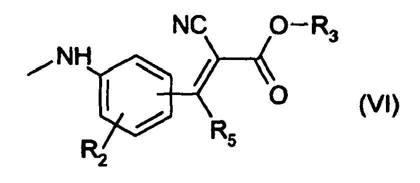 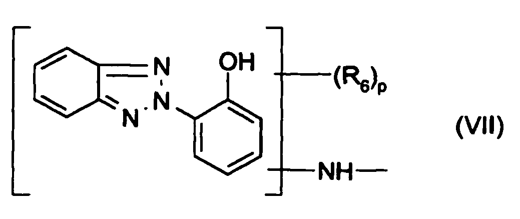 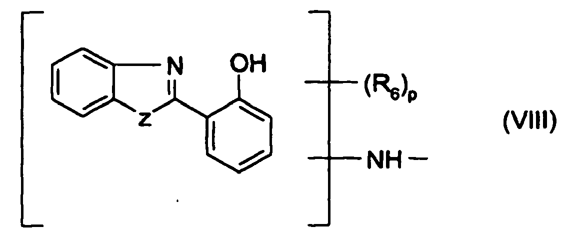 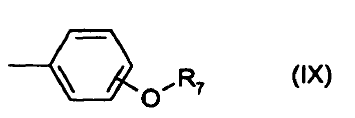 dans lesquelles :
- Xₐ (chacun des Xₐ peut être identique ou différent) représente l'oxygène ou -NH-;
- Rₐ (chacun des Ra peut être identique ou différent) est choisi parmi l'hydrogène; un métal alcalin; un radical ammonium éventuellement substitué par un ou plusieurs radicaux alkyles linéaire ou ramifié en C₁-C₁₈ ou hydroxyalkyles linéaire ou ramifié en C₁-C₁₈; un radical alkyle linéaire ou ramifié en C₁-C_{18,} de préférence en C₆-C₁₂; un radical cycloalkyle en C₁-C₁₂ éventuellement substitué par un ou plusieurs radicaux alkyles en C₁-C₄; un radical polyoxyéthyléné comprenant de 1 à 6 unités d'oxyde d'éthylène et dont le groupe OH terminal est méthylé ; un radical de formule (X), (XI) ou (XII) suivantes : 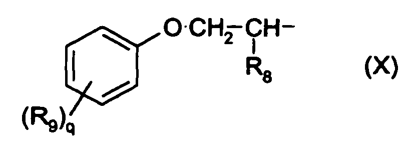 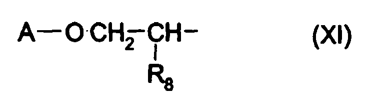 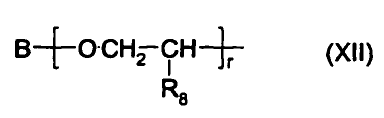
dans lesquelles :
- R₈ est l'hydrogène ou un radical méthyle;
- R₉ est un radical alkyle en C1-C9;
- q est un nombre entier égal à 0; 1; 2; 3;
- r est un nombre entier égal à 1; 2; 3; 4; 5; 6; 7; 8; 9; 10;
- A est un radical alkyle en C₄-C₈ ou un radical cycloalkyl en C₅-C₈;
- B est choisi parmi : un radical alkyle linéaire ou ramifié en C1-C8; un radical cycloalkyle en C₅-C₈; un radical aryle éventuellement substitué par un ou plusieurs radicaux alkyles en C₁-C₄.
- R₁ désigne un radical alkyle en C₃-C₁₈; un radical alcényle en C₂-C₁₈ ; un reste de formule -CH₂-CH(OH)-CH₂-OT₁ où T₁ est un atome d'hydrogène ou un radical alkyle en C₁-C₈; un reste de formule (XIII) suivante : 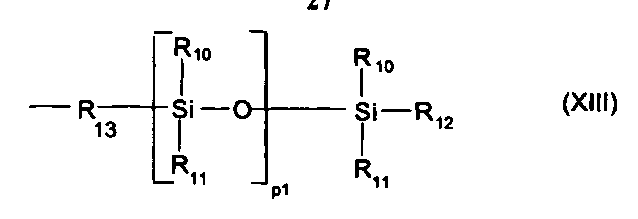
dans laquelle :
- R₁₃ désigne une liaison covalente ; un radical alkyle linéaire ou ramifié en C₁-C₄ ou bien un radical de formule -Cₘ₁H₂ₘ₁-O- où m₁ est un entier égal à 1; 2; 3; 4 ;
- p₁ est un entier égal à 0; 1; 2; 3; 4; 5 ;
- les radicaux R₁₀, R₁₁ et R₁₂, identiques ou différents, désignent un radical alkyle en C₁-C₁₈ ; un radical alcoxy en C₁-C₁₈ ou un radical de formule : 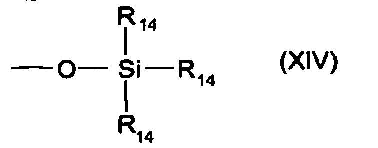
où R₁₄ est un radical alkyle en C₁-C₅.
- R₂ désigne un atome d'hydrogène, un radical alkyle en C1-C4 linéaire ou ramifié ou un radical alkoxy en C1-C4 ;
- R₃ et R₄ , identiques ou différents désignent un radical alkyle linéaire ou ramifié en C₁-C₂₀ ;
- R₅ représente un atome d'hydrogène ou un radical phényle éventuellement substitué par un halogène ou par un radical alkyle en C₁-C₄ ou par un radical alcoxy en C₁-C₄ ;
- R₆ est un radical alkyle en C₁-C₈ linéaire ou ramifié, alkoxy en C1-C3 étant entendu que, dans ce dernier cas, deux R₆ adjacents d'un même noyau aromatique peuvent former ensemble un groupement alkylidène dioxy dans lequel le groupe alkylidène contient de 1 à 2 atomes de carbone, OH, NHCOCH₃ ou NH₂,
- R₇ désigne un atome d'hydrogène, un radical alkyle en C₁-C₁₀, un radical de formule : - (CH₂CHR₅-O)ₙ1R₈ où n₁ est un nombre de 1 à 16, ou bien un radical de structure
- CH₂-CH-(OH)-CH₂OT₁ avec R₈ et T₁ ayant la même signification indiquée ci-dessus.
- Z représente l'oxygène, le soufre, -NH- ou -NR₃ - avec R₃ représentant un radical alkyle en C₁-C₂₀ linéaire ou ramifié;
- p est 0, 1, 2 ou 3,
A₁ peut également être un halogène, un radical -N(R₃)₂, les deux R₃ pouvant former ensemble un cycle de 4 ou 5 atomes de carbone, ou un groupe -OR₃, R₃ ayant la même définition qu'au dessus.

10. Composition selon la revendication 9, où le dérivé de 1,3,5-triazine répond à la formule (I) dans laquelle les A₁, A₂ et A₃ sont de formule (II) et présentant l'ensemble des caractéristiques suivantes :
- l'un des groupements Xₐ-Rₐ représente le radical -NH-Rₐ avec Rₐ choisi parmi : un radical cycloalkyle en C₅-C₁₂ éventuellement substitué par un ou plusieurs radicaux alkyles en C1-C4; un radical de formule (X), (XI) ou (XII) ci-dessus dans lesquelles :
- B est un radical alkyle en C₁-C₄ ;
- R₉ est le radical méthyle ;
- les 2 autres Xₐ-Rₐ représentent le radical -O-Rₐ avec Rₐ, identiques ou différents choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄ ; un radical de formule (X), (XI) ou (XII) ci-dessus dans lesquelles :
- B est un radical alkyle en C₁-C₄;
- R₉ est le radical méthyle.

11. Composition selon la revendication 9, où le dérivé de 1,3,5-triazine répond à la formule (I) dans laquelle les A₁, A₂ et A₃ sont de formule (II) et présentant l'ensemble des caractéristiques suivantes :
- un ou deux Xₐ-Rₐ représente le radical -NH-Rₐ , avec Ra choisi parmi : un radical alkyle linéaire ou ramifié en C₁-C₁₈; un radical cycloalkyle en C₅-C₁₂ éventuellement substitué avec un ou plusieurs radicaux alkyles en C₁-C₄; un radical de formule (X), (XI) ou (XII) ci-dessus dans lesquelles :
- B est un radical alkyle en C₁-C₄ ;
- R9 est le radical méthyle ;
le ou les deux autres Xₐ-Rₐ étant le radical -O-Rₐ avec Rₐ, identiques ou différents choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄; un radical de formule (X), (XI) ou (XII) ci-dessus dans lesquelles :
- B est un radical alkyle en C₁-C₄;
- R₉ est le radical méthyle.

12. Composition selon la revendication 11, où le dérivé de 1,3,5-triazine est la 2-[(p-(tertiobutylamido)anilino]-4,6-bis-[(p-(2'-éthylhexyl-1'-oxycarbonyl)anilino]-1,3,5-triazine de formule suivante : 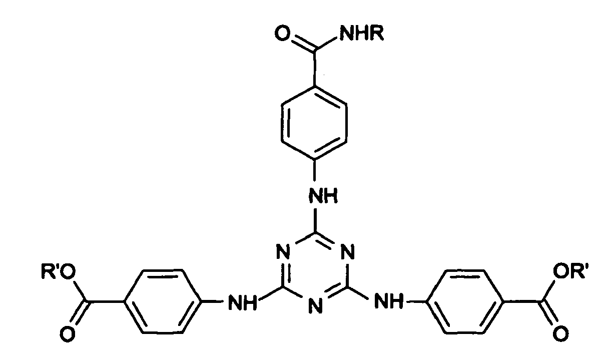 dans laquelle R' désigne un radical éthyl-2 hexyte et R désigne un radical tert-butyle.

13. Composition selon la revendication 9, où le dérivé de 1,3,5-triazine répond à la formule (I) dans laquelle les A₁, A₂ et A₃ sont de formule (II) et présentant l'ensemble des caractéristiques suivantes :
- Xₐ sont identiques et représentent l'oxygène;
- Rₐ, identiques ou différents et représentent un radical alkyle en C₆-C₁₂ ou un radical polyoxyéthyléné comprenant de 1 à 6 unités d'oxyde d'éthylène et dont le groupe OH terminal est méthylé.

14. Composition selon la revendication 13, où le dérivé de 1,3,5-triazine est la 2,4,6-tris[p-(2'-éthylhexyl-1'-oxycarbonyl)anilino]-1,3,5-triazine de formule suivante : 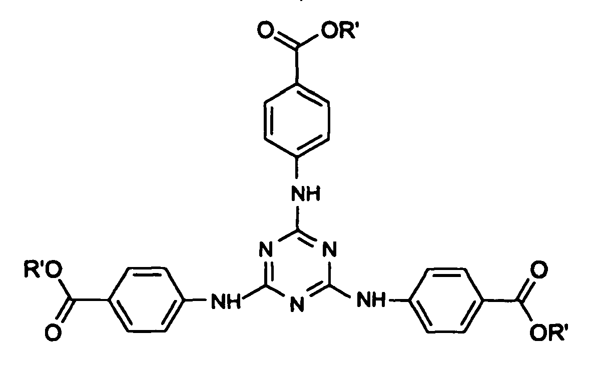 dans laquelle R' désigne un radical 2-éthyl hexyle.

15. Composition selon la revendication 9, où le dérivé de 1,3,5-triazine où le dérivé de 1,3,5-triazine répond à la formule (I) dans laquelle les A₁ et A₂ sont de formule (III) et A₃ est de formule (IX) et présentant l'ensemble des caractéristiques suivantes : R1, identiques ou différents, désignent un radical alkyle en C₃-C₁₈; un radical alcényle en C₂-C₁₈ ou bien un reste de formule -CH₂-CH(OH)-CH₂-OT₁ où T₁ est un atome d'hydrogène ou un radical alkyle en C₁-C₆ ; R₇ désigne un atome d'hydrogène, un radical alkyle en C₁-C₁₀.

16. Composition selon la revendication 15, où le dérivé de 1,3,5-triazine est la 2,4-bis {[4-2-éthyl-hexyloxy)]-2-hydroxy]-phenyl}-6-(4-méthoxy-phenyl)-1,3,5-triazine de formule suivante : 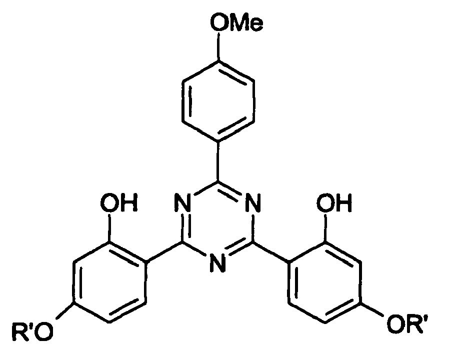 dans laquelle R' désigne un radical éthyl-2 hexyle.

17. Composition selon la revendication 9, où le dérivé de 1,3,5-triazine répond à la formule (I) dans laquelle dans laquelle les A₁, A₂ et A₃ sont de formules (VII) à (XI).

18. Composition selon la revendication 17, où le dérivé de 1,3,5-triazine est choisi parmi :
- la 2,4,6-tris-(4'-amino benzalmalonate de diisobutyle)-s-triazine,
la 2,4,6-tris-(4'-amino benzalmalonate de di(2-éthylhexyle))-s-triazine,
la 2,4,6-tris-(4'-amino benzalmalonate de di(2-éthylhexyle))-6-chloro-s-triazine,
la 2,4,6-tris-(4'-amino benzalmalonate de di(2-éthylhexyle))-6-(4'-aminobenzoate de 2-éthylhexyle)-s-triazine,
la 2,4,6-tris-(4'-amino benzalmalonate de diisobutyle)-6-butoxy-s-triazine,
la 2,4,6-tris-(4'-amino benzalmalonate de diisobutyle)-6-(2-éthylhexylamino)-s-triazine,
la 2,4-bis-(4'-aminobenzylidène camphre)-6-(2-éthylhexyl-amino)-s-triazine,
la 2,4-bis-(4'-aminobenzylidène camphre)-6-(4'-amino benzalmalonate de diisobutyle)-s-triazine,
la 2,4,6-tris(4'-amino benzalmalonate de diéthyle)-s-triazine.
la 2,4,6-tris(4'-amino benzalmalonate de diisopropyle)-s-triazine,
la 2,4,6-tris(4'-amino benzalmalonate de diméthyle)-s-triazine,
la 2,4,6-tris(α-cyano-4-aminocinnamate d'éthyle)-s-triazine.
la 2,4,6-tris[(3'-benzotriazol-2-yl-2'-hydroxy-5'-methyl) phenylamino]-s-triazine,
2,4,6-tris[(3'-benzotriazol-2-yl-2'-hydroxy-5'-ter-octyl) phénylamino]-s-triazine.

19. Composition selon l'une quelconque des revendications 1 à 18, où le ou les dérivés de 1,3,5-triazine sont présents à des concentrations allant de 0,05 à 15% et de préférence de 0,1 à 10 % en poids par rapport au poids total de ladite composition.

20. Composition selon l'une quelconque des revendications 1 à 19, contenant en plus au moins un agent photoprotecteur organique et/ou au moins un agent photoprotecteur minéral complémentaire, hydrosoluble, liposoluble ou insoluble dans les solvants cosmétiques couramment utilisés.

21. Composition selon la revendication 20, où les agents photoprotecteurs organiques complémentaires sont choisis parmi les anthranilates ; les dérivés cinnamiques ; les dérivés salicyliques, les dérivés du camphre; les dérivés de la benzophénone ; les dérivés de β, β'-diphénylacrylate ; les dérivés de benzotriazole ; les dérivés de benzalmalonate ; les dérivés de benzimidazole ; les imadazolines ; les dérivés bis-benzoazolyle ; les dérivés de l'acide p-aminobenzoïque (PABA) ; les dérivés de méthylène bis-(hydroxyphényl benzotriazole) ; les dérivés de benzoxazole ; les polymères filtres et silicones filtres ; les dimères dérivés d'α-alkylstyrène ; les 4,4-diarylbutadiènes et leurs mélanges.

22. Composition selon la revendication 21, où les agents photoprotecteurs organiques complémentaires sont choisis parmi :
Homosalate
Ethylhexyl Salicylate,
Ethylhexyl Methoxycinnamate
Octocrylene,
Phenylbenzimidazole Sulfonic Acid,
Benzophenone-3,
Benzophenone-4,
Benzophenone-5,
2-(4-diethylamino-2-hydroxybenzoyl)-benzoate de n-hexyle.
4-Methylbenzylidene camphor,
Terephthalylidene Dicamphor Sulfonic Acid,
Disodium Phenyl Dibenzimidazole Tetra-sulfonate,
Methylène bis-Benzotriazolyl Tetramethylbutylphénol
Drometrizole Trisiloxane
Polysilicone-15
1,1-dicarboxy (2,2'-diméthyl-propyl)-4,4-diphénylbutadiène
2,4-bis-[5-1(diméthylpropyl)benzoxazol-2-yl-(4-phenyl)-imino]-6-(2-ethykhexy)-imino-1,3,5-triazine
et leurs mélanges.

23. Composition selon la revendication 20, où les agents photoprotecteurs complémentaires minéraux sont choisis parmi des pigments ou des nanopigments d'oxydes métalliques, enrobés ou non.

24. Composition selon la revendication 20, où les filtres complémentaires inorganiques sont des nanopigments d'oxyde de titane, amorphe ou cristallisé, sous forme rutile et/ou anatase, de fer, de zinc, de zirconium ou de cérium

25. Composition selon l'une quelconque des revendications 1 à 24, **caractérisée par le fait qu'**elle comprend en outre au moins un agent de bronzage et/ou de brunissage artificiel de la peau.

26. Composition selon l'une quelconque des revendications 1 à 25, **caractérisée par le fait qu'**elle comprend en outre au moins un adjuvant choisi parmi les corps gras, les solvants organiques, les épaississants ioniques ou non ioniques, hydrophiles ou lipophiles, les adoucissants, les humectants, les opacifiants, les stabilisants, les émollients, les silicones, les agents anti-mousse, les parfums, les conservateurs, les tensioactifs anioniques, cationiques, non-ioniques, zwitterioniques ou amphotères, des actifs, les charges, les polymères, les propulseurs, les agents alcalinisants ou acidifiants

27. Composition selon l'une quelconque des revendications 1 à 25, **caractérisée par le fait que** le mélange huile amidée/dérivé arylalkyl benzoate est présent dans une quantité suffisante permettant de solubiliser à lui-seul la quantité de totale de filtre triazine présent dans la composition.

## Claims

1. Photoprotective cosmetic or dermatological composition, **characterized in that** it comprises:
a) at least one 1,3,5-triazine derivative and
b) a mixture comprising
(i) at least one oil containing in its structure at least one amide unit, and
(ii) at least one arylalkyl benzoate derivative.

2. Composition according to Claim 1, in which the oil containing in its structure at least one amide unit is chosen from the compounds of formula (1) below: 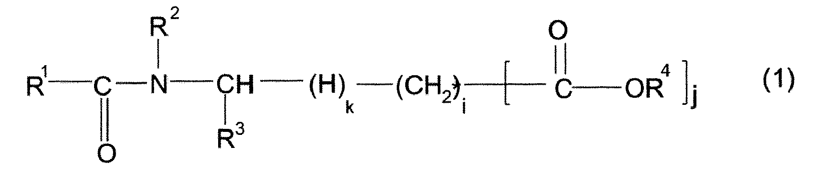 in which:
- the radical R¹ represents an optionally functionalized, aliphatic, cycloaliphatic or cyclic, saturated or unsaturated monovalent hydrocarbon-based radical containing from 1 to 30 carbon atoms and preferably from 1 to 22 carbon atoms, limits inclusive;
- the radicals R², R³ and R⁴, which may be identical or different, represent optionally functionalized, aliphatic, cycloaliphatic or cyclic, saturated or unsaturated monovalent hydrocarbon-based radicals containing from 1 to 30 carbon atoms and preferably from 1 to 22 carbon atoms, limits inclusive;
- k is 0 or 1;
- i is an integer from 0 to 2;
- j is 0 or 1,
- with the proviso that:
- when j = 1, then k is 0 and when j = 0, then i = 0 and k = 1.

3. Composition according to Claim 2, in which the oil containing in its structure at least one amide unit of formula (1) is chosen from those in which:
R¹ represents a linear or branched C₁-C₂₂ alkyl radical;
a linear or branched C₂-C₂₂ alkenyl radical; an aryl radical;
R² represents a hydrogen atom or a linear or branched C₁-C₆ alkyl group;
R³ represents a hydrogen atom or a linear or branched C₁-C₆ alkyl group;
R⁴ represents a linear or branched C₁-C₁₀ alkyl radical or a linear or branched C₂-C₁₀ alkenyl radical or a sterol residue.

4. Composition according to Claim 3, in which the oil containing in its structure at least one amide unit of formula (1) is chosen from:
ethyl N-acetyl-N-butylaminopropionate, having the following formula: 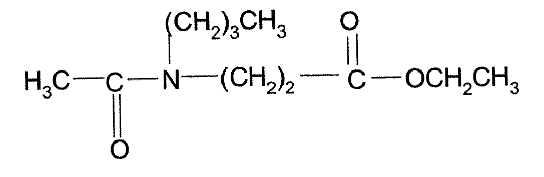
isopropyl N-lauroylsarcosinate of formula: 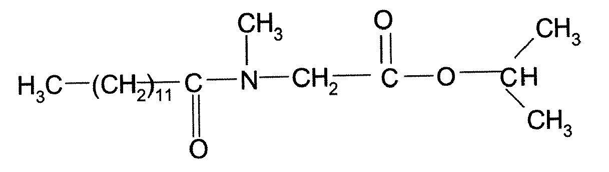
N,N-diethyltoluamide of formula:

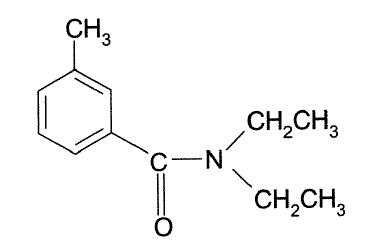

5. Composition according to any one of Claims 1 to 4, in which the oil(s) containing in their structure at least one amide function is (are) present in concentrations preferably ranging from 0.1% to 40% by weight and more preferably from 1% to 20% by weight relative to the total weight of the composition.

6. Composition according to any one of Claims 1 to 5, in which the arylalkyl benzoate derivative is chosen from those of formula (2) or (3) below: 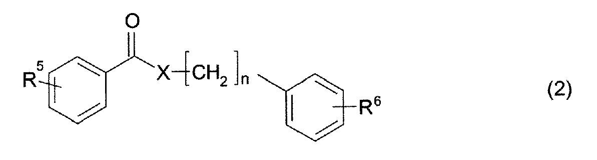 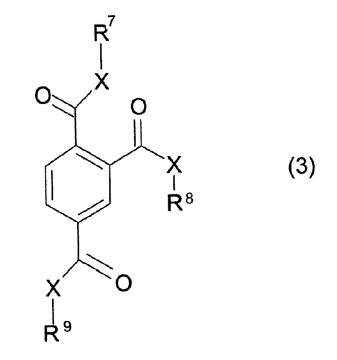 in which:
X denotes O, S or N;
n is an integer from 1 to 10 and more preferably from 2 to 6;
R⁵ and R⁶, which may be identical or different, denote a hydrogen atom; a hydroxyl group; a linear or branched C₁-C₄ alkoxy radical; a nitro radical; an amino radical; a C₆H₆SO₂ radical;
R⁷, R⁸ and R⁹, which may be identical or different, denote a group of formula: 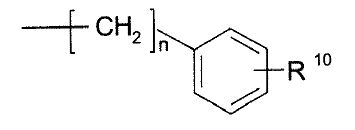
in which n has the same meaning as indicated above; R¹⁰ denotes a hydrogen atom; a hydroxyl group; a linear or branched C₁-C₄ alkoxy radical; a nitro radical; an amino radical; a C₆H₆SO₂ radical.

7. Composition according to Claim 6, in which the arylalkyl benzoate derivative is 2-phenylethyl benzoate of formula:

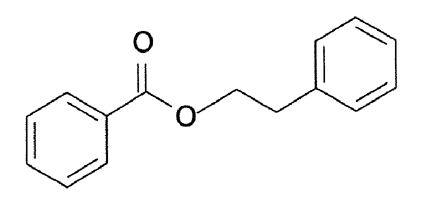

8. Composition according to any one of Claims 1 to 7, in which the alkylaryl benzoate compound is present in concentrations ranging from 0.1% to 40% by weight and more preferably from 1% to 30% by weight relative to the total weight of the composition.

9. Composition according to any one of Claims 1 to 8, in which the 1,3,5-triazine derivative corresponds to formula (I) below: 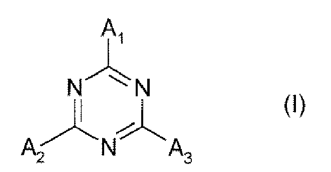 in which the radicals A₁, A₂ and A₃, which may be identical or different, are chosen from the groups of formulae (II) to (IX) below: 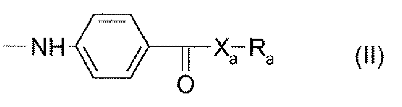 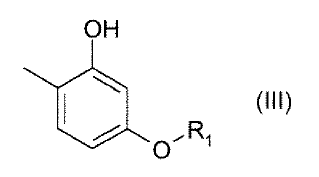 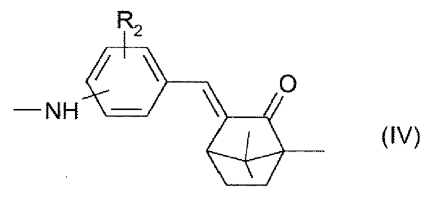 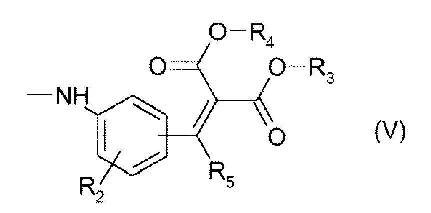 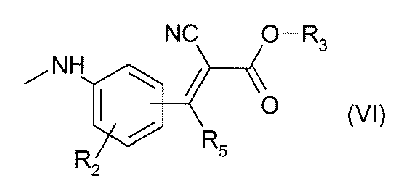 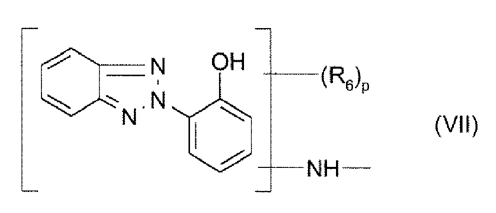 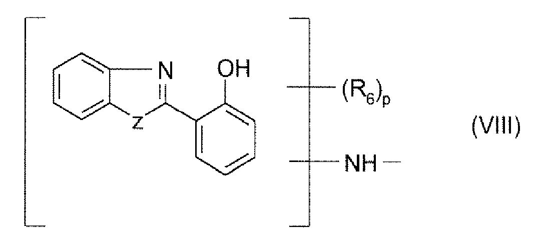 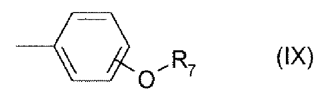 in which:
- Xₐ (each of the groups Xₐ may be identical or different) represents oxygen or -NH-;
- Rₐ (each of the groups Rₐ may be identical or different) is chosen from hydrogen; an alkali metal; an ammonium radical optionally substituted with one or
more linear or branched C₁-C₁₈ alkyl or linear or branched C₁-C₁₈ hydroxyalkyl radicals; a linear or branched C₁-C₁₈ and preferably C₆-C₁₂ alkyl radical; a C₅-C₁₂ cycloalkyl radical optionally substituted with one or more C₁-C₄ alkyl radicals; a polyoxyethylenated radical comprising from 1 to 6 ethylene oxide units, the terminal OH group of which is methylated; a radical of formula (X), (XI) or (XII) below: 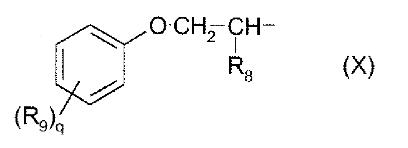 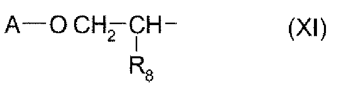 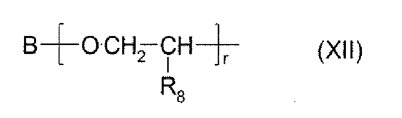 in which:
- R₈ is hydrogen or a methyl radical;
- R₉ is a C₁-C₉ alkyl radical;
- q is an integer equal to 0; 1; 2; 3;
- r is an integer equal to 1; 2; 3; 4; 5; 6; 7; 8; 9; 10;
- A is a C₄-C₈ alkyl radical or a C₅-C₈ cycloalkyl radical;
- B is chosen from: a linear or branched C₁-C₈ alkyl radical; a C₅-C₈ cycloalkyl radical; an aryl radical optionally substituted with one or more C₁-C₄ alkyl radicals;
- R₁ denotes a C₃-C₁₈ alkyl radical; a C₂-C₁₈ alkenyl radical; a residue of formula -CH₂-CH(OH)-CH₂-OT₁ in which T₁ is a hydrogen atom or a C₁-C₈ alkyl radical; a residue of formula (XIII) below: 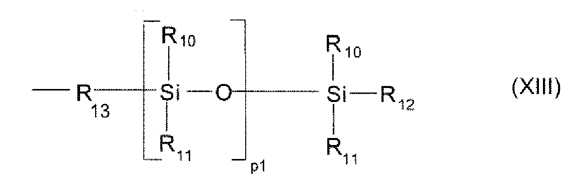
in which:
- R₁₃ denotes a covalent bond; a linear or branched C₁-C₄ alkyl radical or a radical of formula -Cₘ₁H₂ₘ₁-O- in which m₁ is an integer equal to 1; 2; 3; 4;
- p₁ is an integer equal to 0; 1; 2; 3; 4; 5;
- the radicals R₁₀, R₁₁ and R₁₂, which may be identical or different, denote a C₁-C₁₈ alkyl radical; a C₁-C₁₈ alkoxy radical or a radical of formula: 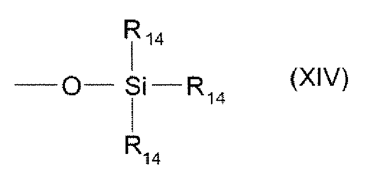
in which R₁₄ is a C₁-C₅ alkyl radical,
- R₂ denotes a hydrogen atom, a linear or branched C₁-C₄ alkyl radical or a C₁-C₄ alkoxy radical;
- R₃ and R₄, which may be identical or different, denote a linear or branched C₁-C₂₀ alkyl radical;
- R₅ represents a hydrogen atom or a phenyl radical optionally substituted with a halogen or with a C₁-C₄ alkyl radical or with a C₁-C₄ alkoxy radical;
- R₆ is a linear or branched C₁-C₈ alkyl radical or a C₁-C₃ alkoxy radical, it being understood that, in the latter case, two adjacent radicals R₆ on the same aromatic nucleus can together form an alkylidenedioxy group in which the alkylidene group contains 1 or 2 carbon atoms, OH, NHCOCH₃ or NH₂,
- R₇ denotes a hydrogen atom, a C₁-C₁₀ alkyl radical, a radical of formula: - (CH₂CHR₅-O)ₙ₁R₈ in which n₁ is a number from 1 to 16, or a radical of structure -CH₂-CH-(OH)-CH₂OT₁ with R₈ and T₁ having the same meaning as indicated above,
- Z represents oxygen, sulfur, -NH- or -NR₃- with R₃ representing a linear or branched C₁-C₂₀ alkyl radical;
- p is 0, 1, 2 or 3,
A₁ can also be a halogen, a radical -N(R₃)₂, the two radicals R₃ together possibly forming a ring of 4 or 5 carbon atoms, or a group -OR₃, R₃ having the same definition as above.

10. Composition according to Claim 9, in which the 1,3,5-triazine derivative corresponds to formula (I) in which A₁, A₂ and A₃ are of formula (II) and have all of the following characteristics:
- one of the groups Xₐ-Rₐ represents a radical-NH-Rₐ with Rₐ chosen from: a C₅-C₁₂ cycloalkyl radical optionally substituted with one or more C₁-C₄ alkyl radicals; a radical of formula (X), (XI) or (XII) above in which:
- B is a C₁-C₄ alkyl radical;
- R₉ is a methyl radical;
- the other two groups Xₐ-Rₐ represent a radical -O-Rₐ with Rₐ, which may be identical or different, chosen from: hydrogen; an alkali metal; an ammonium radical optionally substituted with one or more alkyl or hydroxyalkyl radicals; a linear or branched C₁-C₁₈ alkyl radical; a C₅-C₁₂ cycloalkyl radical optionally substituted with one or more C₁-C₄ alkyl radicals; a radical of formula (X), (XI) or (XII) above in which:
- B is a C₁-C₄ alkyl radical;
- R₉ is a methyl radical.

11. Composition according to Claim 9, in which the 1,3,5-triazine derivative corresponds to formula (I) in which A₁, A₂ and A₃ are of formula (II) and have all of the following characteristics:
- one or two groups Xₐ-Rₐ represent a radical -NH-Rₐ, with Rₐ chosen from: a linear or branched C₁-C₁₈ alkyl radical; a C₅-C₁₂ cycloalkyl radical optionally substituted with one or more C₁-C₄ alkyl radicals; a radical of formula (X), (XI) or (XII) above in which:
- B is a C₁-C₄ alkyl radical;
- R₉ is a methyl radical;
the other or the other two group(s) Xₐ-Rₐ being a radical -O-Rₐ with Rₐ, which may be identical or different, chosen from: hydrogen; an alkali metal; an ammonium radical optionally substituted with one or more alkyl or hydroxyalkyl radicals; a linear or branched C₁-C₁₈ alkyl radical; a C₅-C₁₂ cycloalkyl radical optionally substituted with one or more C₁-C₄ alkyl radicals; a radical of formula (X), (XI) or (XII) above in which:
- B is a C₁-C₄ alkyl radical;
- R₉ is a methyl radical.

12. Composition according to Claim 11, in which the 1,3,5-triazine derivative is 2-[(p-(tert-butylamido)anilino]-4,6-bis[(p-(2'-ethylhexyl-1'-oxycarbonyl)anilino]-1,3,5-triazine of the following formula: 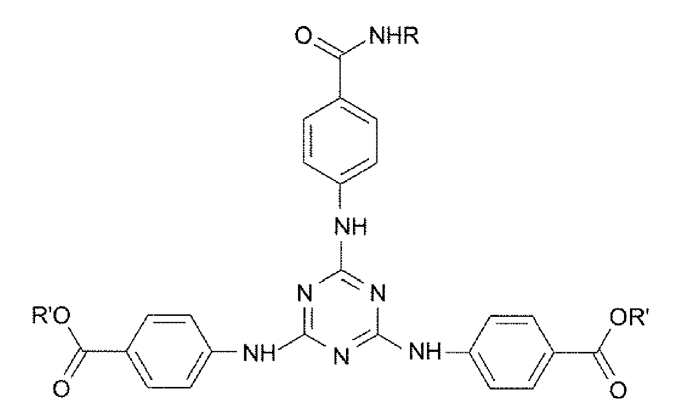 in which R' denotes a 2-ethylhexyl radical and R denotes a tert-butyl radical.

13. Composition according to Claim 9, in which the 1,3,5-triazine derivative corresponds to formula (I) in which A₁, A₂ and A₃ are of formula (II) and have all of the following characteristics:
- Xₐ are identical and represent oxygen;
- Rₐ, which may be identical or different, represent a C₆-C₁₂ alkyl radical or a polyoxyethylenated radical comprising from 1 to 6 ethylene oxide units, the terminal OH group of which is methylated.

14. Composition according to Claim 13, in which the 1,3,5-triazine derivative is 2,4,6-tris[p(2'-ethylhexyl-1'-oxycarbonyl)anilino]-1,3,5-triazine of the following formula: 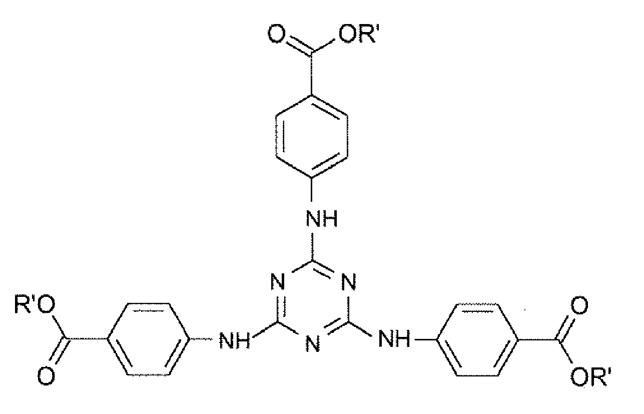 in which R' denotes a 2-ethylhexyl radical.

15. Composition according to Claim 9, in which the 1,3,5-triazine derivative corresponds to formula (I) in which A₁ and A₂ are of formula (III) and A₃ is of formula (IX) and have all of the following characteristics: R₁, which may be identical or different, denote a C₃-C₁₈ alkyl radical; a C₂-C₁₈ alkenyl radical or a residue of formula -CH₂-CH(OH)-CH₂-OT₁ in which T₁ is a hydrogen atom or a C₁-C₈ alkyl radical; R₇ denotes a hydrogen atom or a C₁-C₁₀ alkyl radical.

16. Composition according to Claim 15, in which the 1,3,5-triazine derivative is 2,4-bis{[4-2-ethylhexyloxy)]-2-hydroxy]phenyl}-6-(4-methoxyphenyl)-1,3,5-triazine of the following formula: 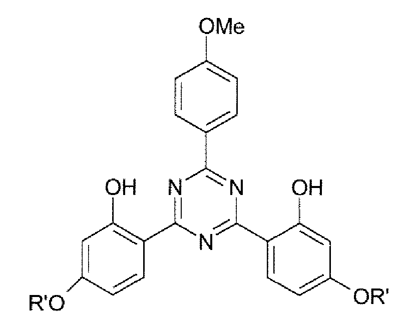 in which R' denotes a 2-ethylhexyl radical.

17. Composition according to Claim 9, in which the 1,3,5-triazine derivative corresponds to formula (I) in which A₁, A₂ and A₃ are of formulae (VII) to (XI).

18. Composition according to Claim 17, in which the 1,3,5-triazine derivative is chosen from:
2,4,6-tris(diisobutyl 4'-aminobenzalmalonate)-s-triazine,
2,4,6-tris(bis(2-ethylhexyl) 4'-aminobenzalmalonate)-s-triazine,
2,4,6-tris(bis(2-ethylhexyl) 4'-aminobenzalmalonate)-6-chloro-s-triazine,
2,4,6-tris(bis(2-ethylhexyl) 4'-aminobenzalmalonate)-6-(2-ethylhexyl 4'-aminobenzoate)-s-triazine, 2,4,6-tris(diisobutyl 4'-aminobenzalmalonate)-6-butoxy-s-triazine,
2,4,6-tris(diisobutyl 4'-aminobenzalmalonate)-6-(2-ethylhexylamino)-s-triazine,
2,4-bis(4'-aminobenzylidenecamphor)-6-(2-ethyl-hexylamino)-s-triazine,
2,4-bis(4'-aminobenzylidenecamphor)-6-(diisobutyl 4'-aminobenzalmalonate)-s-triazine,
2,4,6-tris(diethyl 4'-aminobenzalmalonate)-s-triazine, 2,4,6-tris(diisopropyl 4'-aminobenzalmalonate)-s-triazine,
2,4,6-tris(dimethyl 4'-aminobenzalmalonate)-s-triazine, 2,4,6-tris(ethyl α-cyano-4-aminocinnamate)-s-triazine, 2,4,6-tris[(3'-benzotriazol-2-yl-2'-hydroxy-5'-methyl)phenylamino]-s-triazine,
2,4,6-tris[(3'-benzotriazol-2-yl-2'-hydroxy-5'-tert-octyl)phenylamino]-s-triazine.

19. Composition according to any one of Claims 1 to 18, in which the 1,3,5-triazine derivative(s) is (are) present in concentrations ranging from 0.05% to 15% and preferably from 0.1% to 10% by weight relative to the total weight of the said composition.

20. Composition according to any one of Claims 1 to 19, also containing at least one additional organic photoprotective agent and/or at least one additional mineral photoprotective agent, which is water-soluble, liposoluble or insoluble in the cosmetic solvents commonly used.

21. Composition according to Claim 20, in which the additional organic photoprotective agents are chosen from anthranilates; cinnamic derivatives; salicylic derivatives; camphor derivatives; benzophenone derivatives; β,β'-diphenylacrylate derivatives; benzotriazole derivatives; benzalmalonate derivatives; benzimidazole derivatives; imidazolines; bis-benzazolyl derivatives; p-aminobenzoic acid (PABA) derivatives; methylenebis(hydroxyphenylbenzotriazole) derivatives; benzoxazole derivatives; screening polymers and screening silicones; dimers derived from α-alkylstyrene; 4,4-diarylbutadienes, and mixtures thereof.

22. Composition according to Claim 21, in which the additional organic photoprotective agents are chosen from:
Homosalate,
Ethylhexyl salicylate,
Ethylhexyl methoxycinnamate,
Octocrylene,
Phenylbenzimidazolesulfonic acid,
Benzophenone-3,
Benzophenone-4,
Benzophenone-5,
n-Hexyl 2-(4-diethylamino-2-hydroxybenzoyl)benzoate, 4-Methylbenzylidenecamphor,
Terephthalylidenedicamphorsulfonic acid,
Disodium phenyldibenzimidazoletetrasulfonate, Methylenebis(benzotriazolyl)tetramethylbutylphenol, Drometrizole trisiloxane,
Polysilicone-15,
1,1-Dicarboxy(2,2'-dimethylpropyl)-4,4-diphenyl-butadiene,
2,4-Bis[5-1(dimethylpropyl)benzoxazol-2-yl(4-phenyl)imino]-6-(2-ethylhexyl)imino-1,3,5-triazine, and mixtures thereof.

23. Composition according to Claim 20, in which the additional mineral photoprotective agents are chosen from coated or uncoated metal oxide pigments or nanopigments.

24. Composition according to Claim 20, in which the additional mineral screening agents are nanopigments of titanium oxide, which is amorphous or crystallized in rutile and/or anatase form, or of iron oxide, zinc oxide, zirconium oxide or cerium oxide.

25. Composition according to any one of Claims 1 to 24, **characterized in that** it also comprises at least one agent for artificially tanning and/or browning the skin.

26. Composition according to any one of Claims 1 to 25, **characterized in that** it also comprises at least one adjuvant chosen from fatty substances, organic solvents, ionic or nonionic, hydrophilic or lipophilic thickeners, softeners, humectants, opacifiers, stabilizers, emollients, silicones, antifoams, fragrances, preserving agents, anionic, cationic, nonionic, zwitterionic or amphoteric surfactants, active agents, fillers, polymers, propellants and acidifying or basifying agents.

27. Composition according to any one of Claims 1 to 25, **characterized in that** the amide-based oil/arylalkyl benzoate derivative mixture is present in an amount that is sufficient for dissolving by itself all of the triazine screening agent present in the composition.

## Patentansprüche

1. Kosmetische oder dermatologische Lichtschutzzusammensetzung, **dadurch gekennzeichnet, dass** sie
a) mindestens ein 1,3,5-Triazinderivat und
b) eine Mischung, umfassend
(i) mindestens ein Öl, das in seiner Struktur mindestens eine Amideinheit aufweist, und
(ii) mindestens ein Arylalkylbenzoatderivat, umfasst.

2. Zusammensetzung nach Anspruch 1, wobei das Öl in seiner Struktur mindestens eine Amideinheit aufweist, die unter den Verbindungen der folgenden Formel (1) ausgewählt ist: 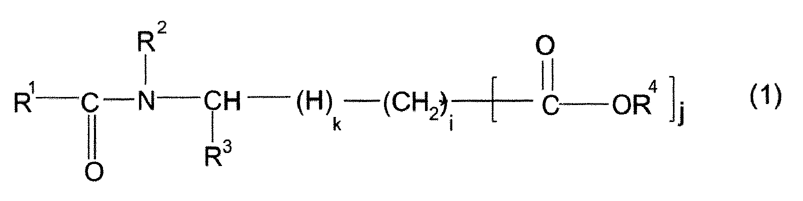 worin:
- der Rest R¹ für einen gegebenenfalls funktionalisierten, aliphatischen, cycloaliphatischen oder cyclischen, gesättigten oder ungesättigten, einwertigen Kohlenwasserstoffrest mit 1 bis 30 Kohlenstoffatomen und vorzugsweise 1 bis 22 Kohlenstoffatomen einschließlich der Grenzwerte steht;
- die Reste R², R³ und R⁴ gleich oder verschieden sein können und für Wasserstoff oder gegebenenfalls funktionalisierten, aliphatischen, cycloaliphatischen oder cyclischen, gesättigten oder ungesättigten, einwertigen Kohlenwasserstoffrest mit 1 bis 30 Kohlenstoffatomen und vorzugsweise 1 bis 22 Kohlenstoffatomen einschließlich der Grenzwerte stehen;
- k für 0 oder 1 steht;
- i für eine ganze Zahl von 0 bis 2 steht;
- j für 0 oder 1 steht;
- mit der Maßgabe, dass:
- dann, wenn j = 1, k für 0 steht, und dann, wenn j = 0, k = 1.

3. Zusammensetzung nach Anspruch 2, wobei das Öl in seiner Struktur mindestens eine Amideinheit der Formel (1) aufweist und unter denjenigen ausgewählt ist, worin:
R¹ für einen linearen oder verzweigten C₁-C₂₂-Alkylrest; einen linearen oder verzweigten C₂-C₂₂-Alkenylrest oder einen Arylrest steht;
R² für ein Wasserstoffatom oder eine lineare oder verzweigte C₁-C₆-Alkylgruppe steht;
R³ für ein Wasserstoffatom oder eine lineare oder verzweigte C₁-C₆-Alkylgruppe steht;
R⁴ für einen linearen oder verzweigten C₁-C₁₀-Alkylrest; einen linearen oder verzweigten C₂-C₁₀-Alkenylrest oder einen Sterolrest steht.

4. Zusammensetzung nach Anspruch 3, wobei das Öl in seiner Struktur mindestens eine Amideinheit der Formel (1) aufweist und unter N-Acetyl-N-butylaminopropionsäureethylester der folgenden Formel: 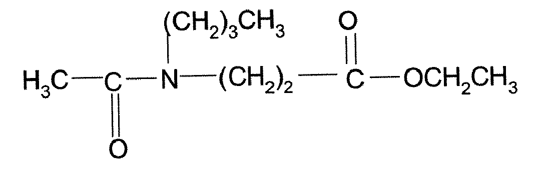 N-Lauroylsarkosinisopropylester der Formel: 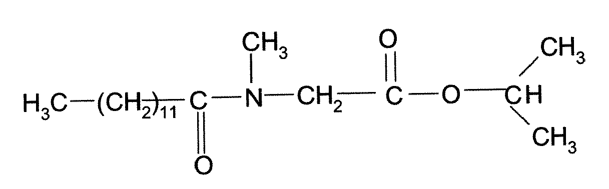 N,N-Diethyltoluamid der Formel: 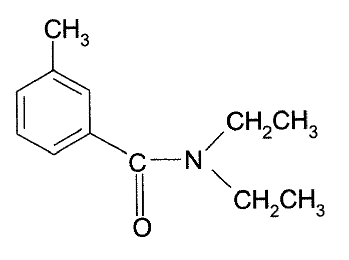 ausgewählt ist.

5. Zusammensetzung nach einem der Ansprüche 1 bis 4, wobei das Öl bzw. die Öle, das bzw. die in seiner bzw. ihrer Struktur mindestens eine Amidfunktion aufweist bzw. aufweisen, in Konzentrationen von vorzugsweise 0,1 bis 40 Gew.-% und weiter bevorzugt 1 bis 20 Gew.-%, bezogen auf das Gesamtgewicht der Zusammensetzung, vorliegt bzw. vorliegen.

6. Zusammensetzung nach einem der Ansprüche 1 bis 5, wobei das Arylalkylbenzoatderivat unter denjenigen der folgenden Formel (2) oder (3) ausgewählt ist: 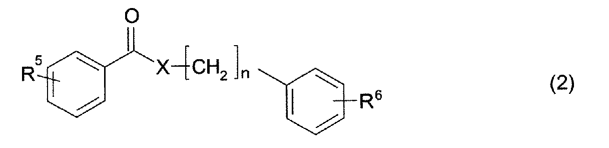 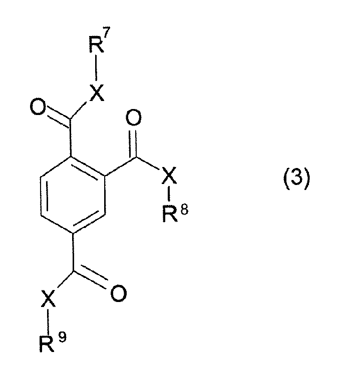 worin:
X für O, S oder N steht;
n für eine ganze Zahl von 1 bis 10 und vorzugsweise 2 bis 6 steht;
R⁵ und R⁶ gleich oder verschieden sind und für ein Wasserstoffatom; eine Hydroxylgruppe; einen linearen oder verzweigten C₁-C₄-Alkoxyrest; einen Nitrorest, einen Aminorest oder einen C₆H₆SO₂-Rest stehen;
R⁷, R⁸ und R⁹ gleich oder verschieden sind und für eine Gruppe der Formel: 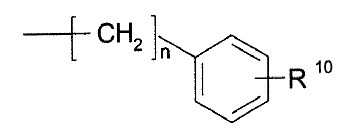
stehen, wobei n die oben angegebene Bedeutung besitzt; und R¹⁰ für ein Wasserstoffatom; eine Hydroxylgruppe; einen linearen oder verzweigten C₁-C₄-Alkoxyrest; einen Nitrorest, einen Aminorest oder einen C₆H₆SO₂-Rest steht.

7. Zusammensetzung nach Anspruch 6, wobei es sich bei dem Arylalkylbenzoatderivat um 2-Phenylethylbenzoat der Formel: 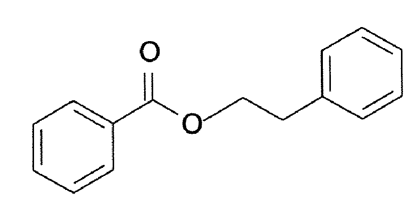 handelt.

8. Zusammensetzung nach einem der Ansprüche 1 bis 7, wobei die Alkylarylbenzoatverbindung in Konzentrationen von 0,1 bis 40 Gew.-% und weiter bevorzugt 1 bis 30 Gew.-%, bezogen auf das Gesamtgewicht der Zusammensetzung, vorliegt.

9. Zusammensetzung nach einem der Ansprüche 1 bis 8, wobei das 1,3,5-Triazinderivat der folgenden Formel (I) entspricht: 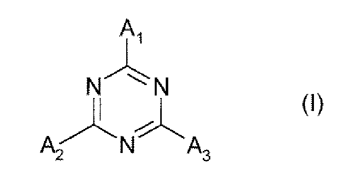 worin die Reste A₁, A₂ und A₃ gleich oder verschieden sind und unter den Gruppen der folgenden Formeln (II) bis (IX) ausgewählt sind: 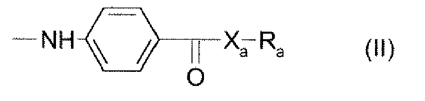 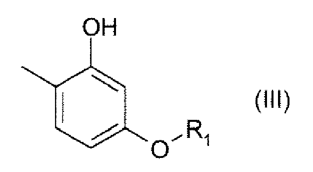 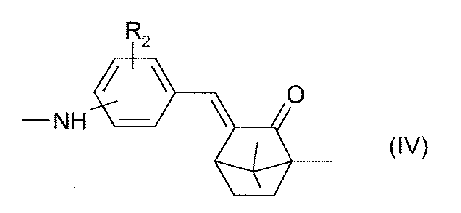 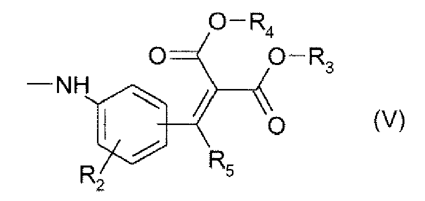 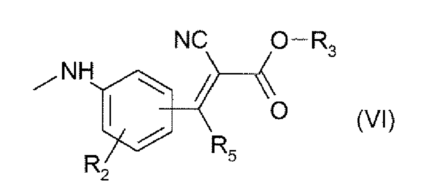 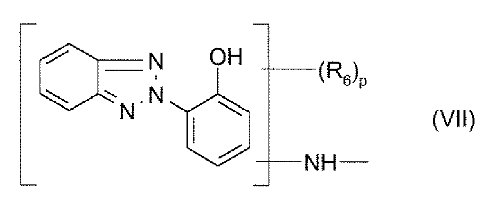 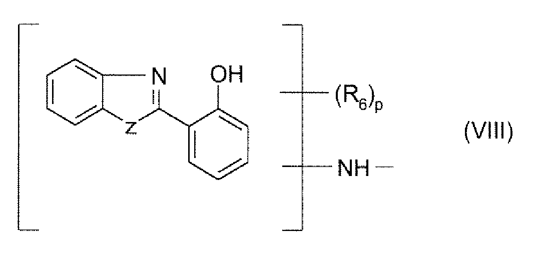 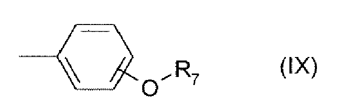 worin:
- Xₐ (wobei jede der Gruppen Xₐ gleich oder verschieden sein kann) für Sauerstoff oder -NHsteht;
- Rₐ (wobei jede der Gruppen Rₐ gleich oder verschieden sein kann) unter Wasserstoff; einem Alkalimetall; einem Ammoniumrest, der gegebenenfalls durch einen oder mehrere lineare oder verzweigte C₁-C₁₈-Alkylreste oder einen oder mehrere lineare oder verzweigte C₁-C₁₈-Hydroxyalkylreste substituiert ist; einem linearen oder verzweigten C₁-C₁₈-Alkylrest, vorzugsweise C₆-C₁₂-Alkylrest; einem C₅-C₁₂-Cycloalkylrest, der gegebenenfalls durch einen oder mehrere C₁-C₄-Alkylreste substituiert ist; einem Polyoxyethylenrest mit 1 bis 6 Ethylenoxid-Einheiten und methylierter endständiger OH-Gruppe und einem Rest der folgenden Formel (X), (XI) oder (XII) ausgewählt ist: 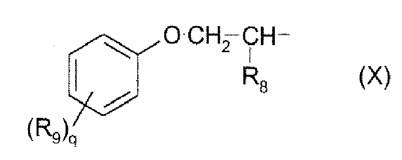 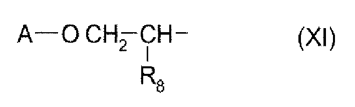 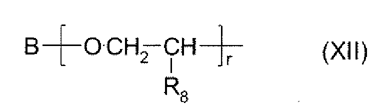
worin:
- R₈ für Wasserstoff oder einen Methylrest steht;
- R₉ für einen C₁-C₉-Alkylrest steht;
- q für eine ganze Zahl mit einem Wert von 0, 1, 2 oder 3 steht;
- r für eine ganze Zahl mit einem Wert von 1, 2, 3, 4, 5, 6, 7, 8, 9 oder 10 steht;
- A für einen C₄-C₈-Alkylrest oder einen C₅-C₈-Cycloalkylrest steht;
- B unter einem linearen oder verzweigten C₁-C₈-Alkylrest; einem C₅-C₈-Cycloalkylrest und einem Arylrest, der gegebenenfalls durch einen oder mehrere C₁-C₄-Alkylreste substituiert ist; ausgewählt ist;
- R₁ für einen C₃-C₁₈-Alkylrest; einen C₂-C₁₈-Alkenylrest; einen Rest der Formel -CH₂-CH(OH)-CH₂-OT₁, worin T₁ für ein Wasserstoffatom oder einen C₁-C₈-Alkylrest steht; oder einen Rest der folgenden Formel (XIII) steht: 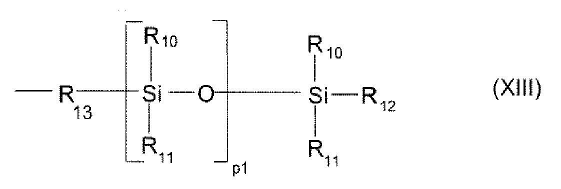
worin:
- R₁₃ für eine kovalente Bindung; einen linearen oder verzweigten C₁-C₄-Alkylrest oder auch einen Rest der Formel -Cₘ₁H₂ₘ₁-O-, worin m₁ für eine ganze Zahl mit einem Wert von 1, 2, 3 oder 4 steht; steht;
- p₁ für eine ganze Zahl mit einem Wert von 0, 1, 2, 3, 4 oder 5 steht;
- die Reste R₁₀, R₁₁ und R₁₂ gleich oder verschieden sind und für einen C₁-C₁₈-Alkylrest; einen C₁-C₁₈-Alkoxyrest oder einen Rest der Formel: 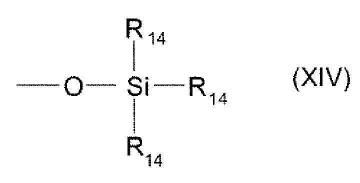
stehen, wobei R₁₄ für einen C₁-C₅-Alkylrest steht;
- R² für ein Wasserstoffatom, einen linearen oder verzweigten C₁-C₄-Alkylrest oder einen C₁-C₄-Alkoxyrest stehen;
- R₃ und R₄ gleich oder verschieden sind und für einen linearen oder verzweigten C₁-C₂₀-Alkylrest stehen;
- R₅ für ein Wasserstoffatom oder einen Phenylrest, der gegebenenfalls durch ein Halogen oder durch einen C₁-C₄-Alkylrest oder durch einen C₁-C₄-Alkoxyrest substituiert ist, steht;
- R₆ für einen linearen oder verzweigten C₁-C₈-Alkylrest oder einen C₁-C₃-Alkoxyrest steht, mit der Maßgabe, dass in letzterem Fall zwei benachbarte Reste R₆ am selben aromatischen Kern zusammen eine Alkylidendioxygruppe bilden können, wobei die Alkylidengruppe 1 bis 2 Kohlenstoffatome, OH, NHCOCH₃ oder NH₂ enthält;
- R₇ für ein Wasserstoffatom, einen C₁-C₁₀-Alkylrest, einen Rest der Formel -(CH₂CHR₅-O)ₙ₁R₈, wobei n₁ für eine Zahl von 1 bis 16 steht, oder einen Rest der Struktur -CH₂-CH-(OH)-CH2OT₁, wobei R₈ und T₁ die oben angegebene Bedeutung besitzen, steht;
- Z für Sauerstoff, Schwefel, -NH- oder -NR₃-, wobei R₃ für einen linearen oder verzweigten C₁-C₂₀-Alkylrest steht, steht;
- p für 0, 1, 2 oder 3 steht;
A₁ auch für ein Halogen, einen Rest -N(R₃)2, wobei die beiden Reste R₃ zusammmen einen Ring mit 4 oder 5 Kohlenstoffatomen bilden können, oder eine Gruppe -OR₃, wobei R₃ die oben angegebene Bedeutung besitzt, stehen kann.

10. Zusammensetzung nach Anspruch 9, wobei das 1,3,5-Triazinderivat der Formel (I) entspricht, worin A₁, A₂ und A₃ der Formel (II) entsprechen und alle der folgenden Merkmale aufweisen:
- eine der Gruppen Xₐ-Rₐ steht für einen Rest -NH-Rₐ, wobei Rₐ unter einem C₅-C₁₂-Cycloalkylrest, der gegebenenfalls durch einen oder mehrere C₁-C₄-Alkylreste substituiert ist; einem Rest der Formel (X), (XI) oder (XII) oben, worin
- B für einen C₁-C₄-Alkylrest steht;
- R₉ für einen Methylrest steht;
ausgewählt ist;
- die 2 anderen Xₐ-Rₐ stehen für einen Rest -O-Rₐ, wobei die Reste Rₐ gleich oder verschieden sind und unter Wasserstoff; einem Alkalimetall; einem Ammoniumrest, der gegebenenfalls durch einen oder mehrere Alkyl- oder Hydroxyalkylreste substituiert ist; einem linearen oder verzweigten C₁-C₁₈-Alkylrest; einem C₅-C₁₂-Cycloalkylrest, der gegebenenfalls durch einen oder mehrere C₁-C₄-Alkylreste substituiert ist; einem Rest der Formel (X), (XI) oder (XII) oben, worin
- B für einen C₁-C₄-Alkylrest steht;
- R₉ für einen Methylrest steht;
ausgewählt sind.

11. Zusammensetzung nach Anspruch 9, wobei das 1,3,5-Triazinderivat der Formel (I) entspricht, worin A₁, A₂ und A₃ der Formel (II) entsprechen und alle der folgenden Merkmale aufweisen:
- eine oder zwei Gruppen Xₐ-Rₐ stehen für einen Rest -NH-Rₐ, wobei Rₐ unter einem linearen oder verzweigten C₁-C₁₈-Alkylrest; einem C₅-C₁₂-Cycloalkylrest, der gegebenenfalls durch einen oder mehrere C₁-C₄-Alkylreste substituiert ist; einem Rest der Formel (X), (XI) oder (XII) oben, worin
- B für einen C₁-C₄-Alkylrest steht;
- R₉ für einen Methylrest steht;
ausgewählt ist;
- der bzw. die beiden anderen Gruppen Xₐ-Rₐ stehen für einen Rest -O-Rₐ, wobei die Reste Rₐ gleich oder verschieden sind und unter Wasserstoff; einem Alkalimetall; einem Ammoniumrest, der gegebenenfalls durch einen oder mehrere Alkyl - oder Hydroxyalkylreste substituiert ist; einem linearen oder verzweigten C₁-C₁₈-Alkylrest; einem C₅-C₁₂-Cycloalkylrest, der gegebenenfalls durch einen oder mehrere C₁-C₄-Alkylreste substituiert ist; einem Rest der Formel (X), (XI) oder (XII) oben, worin
- B für einen C₁-C₄-Alkylrest steht;
- R₉ für einen Methylrest steht;
ausgewählt sind.

12. Zusammensetzung nach Anspruch 11, wobei es sich bei dem 1,3,5-Triazinderivat um 2-[(p-(tert.-Butylamido)anilino]-4,6-bis[(p-(2'-ethylhexyl-1'-oxycarbonyl)anilino]-1,3,5-triazin der folgenden Formel handelt: 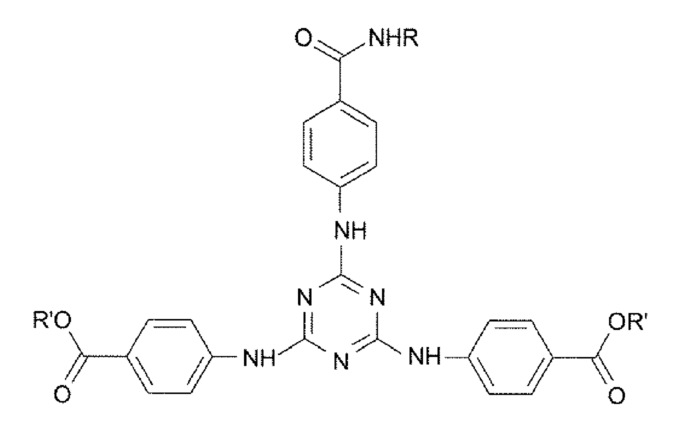 worin R' für einen 2-Ethylhexylrest steht und R für einen tert.-Butylrest steht.

13. Zusammensetzung nach Anspruch 9, wobei das 1,3,5-Triazinderivat der Formel (I) entspricht, worin A₁, A₂ und A₃ der Formel (II) entsprechen und alle der folgenden Merkmale aufweisen:
- die Reste Xₐ sind gleich und stehen für Sauerstoff;
- die Reste Rₐ sind gleich oder verschieden und stehen für einen C₆-C₁₂-Alkylrest oder einen Polyoxyethylenrest mit 1 bis 6 Ethylenoxid-Einheiten und methylierter endständiger OH-Gruppe.

14. Zusammensetzung nach Anspruch 13, wobei es sich bei dem 1,3,5-Triazinderivat um 2,4,6-Tris[p-(2'-ethylhexyl-1'-oxycarbonyl)anilino]-1,3,5-triazin der folgenden Formel handelt: 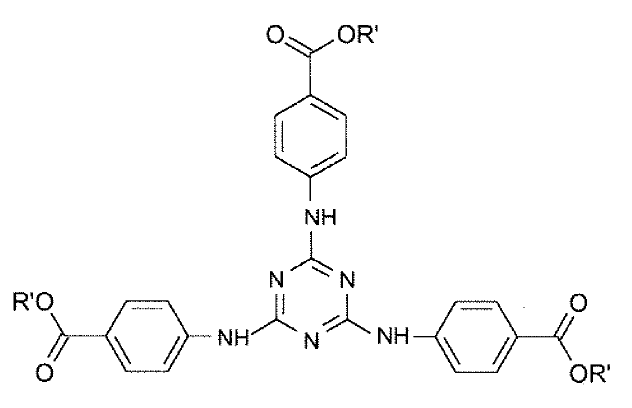 worin R' für einen 2-Ethylhexylrest steht.

15. Zusammensetzung nach Anspruch 9, wobei das 1,3,5-Triazinderivat der Formel (I) entspricht, worin A₁ und A₂ der Formel (III) entsprechen und A₃ der Formel (IX) entspricht und alle der folgenden Merkmale aufweisen: die Reste R₁ sind gleich oder verschieden und stehen für einen C₃-C₁₈-Alkylrest; einen C₂-C₁₈-Alkenylrest oder einen Rest der Formel -CH₂-CH(OH)-CH₂-OT₁, worin T₁ für ein Wasserstoffatom oder einen C₁-C₈-Alkylrest steht; R₇ steht für ein Wasserstoffatom oder einen C₁-C₁₀-Alkylrest.

16. Zusammensetzung nach Anspruch 15, wobei es sich bei dem 1,3,5-Triazinderivat um 2,4-Bis{[4-2-ethylhexyloxy)]-2-hydroxy]phenyl}-6-(4-methoxyphenyl)-1,3,5-triazin der folgenden Formel handelt: 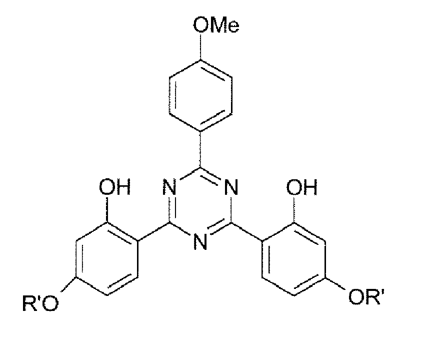 worin R' für einen 2-Ethylhexylrest steht.

17. Zusammensetzung nach Anspruch 9, wobei das 1,3,5-Triazinderivat der Formel (I) entspricht, worin die Gruppen A₁, A₂ und A₃ den Formeln (VII) bis (XI) entsprechen.

18. Zusammensetzung nach Anspruch 17, wobei das 1,3,5-Triazinderivat unter
2,4,6-Tris(diisobutyl-4'-aminobenzalmalonat)-s-triazin,
2,4,6-Tris(di(2-ethylhexyl)-4'-aminobenzalmalonat)-s-triazin,
2,4,6-Tris(di(2-ethylhexyl)-4'-aminobenzalmalonat)-6-chlor-s-triazin,
2,4,6-Tris(di(2-ethylhexyl)-4'-aminobenzalmalonat)-6-(2-ethylhexyl-4'-aminobenzoat)-s-triazin,
2,4,6-Tris(diisobutyl-4'-aminobenzalmalonat)-6-butoxy-s-triazin,
2,4,6-Tris(diisobutyl-4'-aminobenzalmalonat)-6-(2-ethylhexylamino)-s-triazin,
2,4-Bis(4'-aminobenzylidencampher)-6-(2-ethylhexylamino)-s-triazin,
2,4-Bis(4'-aminobenzylidencampher)-6-(diisobutyl-4'-aminobenzalmalonat)-s-triazin,
2,4,6-Tris(diethyl-4'-aminobenzalmalonat)-s-triazin,
2,4,6-Tris(diisopropyl-4'-aminobenzalmalonat)-s-triazin,
2,4,6-Tris(dimethyl-4'-aminobenzalmalonat)-s-triazin,
2,4,6-Tris(ethyl-α-cyano-4-aminocinnamat)-s-triazin,
2,4,6-Tris[(3'-benzotriazol-2-yl-2'-hydroxy-5'-methyl)phenylamino]-s-triazin,
2,4,6-Tris[(3'-benzotriazol-2-yl-2'-hydroxy-5'-tert.-octyl)phenylamino]-s-triazin
ausgewählt ist.

19. Zusammensetzung nach einem der Ansprüche 1 bis 18, wobei das 1,3,5-Triazinderivat bzw. die 1,3,5-Triazinderivate in Konzentrationen von 0,05 bis 15 Gew.-% und vorzugsweise 0,1 bis 10 Gew.-%, bezogen auf das Gesamtgewicht der Zusammensetzung, vorliegt bzw. vorliegen.

20. Zusammensetzung nach einem der Ansprüche 1 bis 19, die außerdem mindestens ein zusätzliches organisches Lichtschutzmittel und/oder mindestens ein zusätzliches anorganisches Lichtschutzmittel, das wasserlöslich, fettlöslich oder in den gemeinhin verwendeten kosmetischen Lösungsmitteln unlöslich ist, enthält.

21. Zusammensetzung nach Anspruch 20, wobei die zusätzlichen organischen Lichtschutzmittel unter Anthranilaten; Zimtsäurederivaten; Salicylsäurederivaten, Campherderivaten; Benzophenonderivaten; β,β'-Diphenylacrylatderivaten; Benzotriazolderivaten; Benzalmalonatderivaten; Benzimidazolderivaten; Imidazolinen; Bisbenzazolylderivaten; p-Aminobenzoesäurederivaten (PABA-Derivaten); Methylenbis(hydroxyphenylbenzotriazol)derivaten; Benzoxazolderivaten; Filterpolymeren und Filtersilikonen; von α-Alkylstyrol abgeleiteten Dimeren; 4,4-Diarylbutadienen und Mischungen davon ausgewählt sind.

22. Zusammensetzung nach Anspruch 21, wobei die zusätzlichen organischen Lichtschutzmittel unter Homosalate,
Ethylhexyl Salicylate,
Ethylhexyl Methoxycinnamate,
Octocrylene,
Phenylbenzimidazole Sulfonic Acid,
Benzophenone-3,
Benzophenone-4,
Benzophenone-5,
2-(4-Diethylamino-2-hydroxybenzoyl)benzoesäure-n-hexylester,
4-Methylbenzylidene camphor,
Terephthalylidene Dicamphor Sulfonic Acid, Disodium Phenyl Dibenzimidazole Tetrasulfonate, Methylenbis(benzotriazolyl)tetramethylbutylphenol, Drometrizole Trisiloxane, Polysilicone-15,
1,1-Dicarboxy(2,2'-dimethylpropyl)-4,4-diphenylbutadien,
2,4-Bis[5-1(dimethylpropyl)benzoxazol-2-yl(4-phenyl)imino]-6-(2-ethylhexyl)imino-1,3,5-triazin und Mischungen davon ausgewählt sind.

23. Zusammensetzung nach Anspruch 20, wobei es sich bei den zusätzlichen anorganischen Lichtschutzmitteln um beschichtete oder unbeschichtete Metalloxidpigmente oder -nanopigmente handelt.

24. Zusammensetzung nach Anspruch 20, wobei es sich bei den zusätzlichen anorganischen Filtern um Nanopigmente aus Titanoxid, das amorph oder kristallin in Rutil- und/oder Anatasform ist, Eisenoxid, Zinkoxid, Zirconiumoxid oder Ceroxid handelt.

25. Zusammensetzung nach einem der Ansprüche 1 bis 24, **dadurch gekennzeichnet, dass** sie außerdem mindestens ein Mittel zur künstlichen Bräunung und/oder Braunfärbung der Haut umfasst.

26. Zusammensetzung nach einem der Ansprüche 1 bis 25, **dadurch gekennzeichnet, dass** sie außerdem mindestens einen Hilfsstoff, der unter Fettsubstanzen, organischen Lösungsmitteln, ionischen oder nichtionischen, hydrophilen oder lipophilen Verdickungsmitteln, zartmachenden Mitteln, Feuchtigkeitsspendern, Trübungsmitteln, Stabilisatoren, Emollientien, Silikonen, Schaumhemmern, Duftstoffen, Konservierungsmitteln, anionischen, kationischen, nichtionischen, zwitterionischen oder amphoteren Tensiden, Wirkstoffen, Füllstoffen, Polymeren, Treibmitteln, Alkalinisierungsmitteln oder Ansäuerungsmitteln ausgewählt ist, umfasst.

27. Zusammensetzung nach einem der Ansprüche 1 bis 25, **dadurch gekennzeichnet, dass** die Mischung aus amidgruppenhaltigem Öl und Arylalkylbenzoatderivat in einer Menge vorliegt, die für sich alleine zur Solubilisierung des gesamten in der Zusammensetzung vorliegenden Triazinfilters ausreicht.
